# EUROPEAN PATENT APPLICATION

(11) **EP 2 189 541 A2**
(43) Date of publication of application: **26.05.2010**
(21) Application number: 10150252.4
(22) Date of filing: 23.08.2006
(51) Int. Cl.: C12Q 1/68

(54) **Methods and reagents for treatment and diagnosis of age-related macular degeneration**

(30) Priority: 09.09.2005 US 715503 P
(62) Divisional of application: 06813703.3
(71) Applicant: University of Iowa Research Foundation, Iowa City, IA 52242-5500 (US)
(72) Inventor: Hageman, Gregory, S., Coralville, CA 52241 (US)
(74) Representative: Evenson, Jane Harriet

(57) **Abstract**

The invention relates to proteins associated with age-related macular degeneration (AMD). These proteins, which are present in blood, are expressed in individuals with AMD at either elevated or reduced levels compared to healthy individuals. The invention provides methods for diagnosing AMD. The invention provides methods for assessing the efficacy of treatment of AMD. The invention provides methods for monitoring the progression of AMD.

## Description

### BACKGROUND OF THE INVENTION

Pathological changes associated with many disease states are reflected in the protein profile of serum and plasma (because blood comes into contact with most of the tissues in the human body) as well as other body fluids. Monitoring the levels (and changes in levels) of such proteins, or "biomarkers" is useful for diagnosis and prognosis of diseases. In addition, changes in levels of biomarker can serve as surrogate endpoints for assessing the effects and efficacy of therapeutic interventions.

Age-related macular degeneration (AMD) is a degenerative condition of a specialized region of the central retina called the macula. AMD is the leading cause of blindness in adults over 60, affecting more than 50 million people worldwide (Klein et al., Am J Ophthalmol. 137:486, 2004). Although some therapeutic options are available for patients with AMD, and others are being developed, there is a great need for additional treatments. Similarly, there is a great need for new methods for diagnosis of AMD, and for prognosis of AMD patients. The present invention addresses these and other needs.

### BRIEF SUMMARY OF THE INVENTION

The invention relates to proteins associated with age-related macular degeneration (AMD). These AMD-associated proteins (biomarkers) are differentially present in the serum or blood fraction of individuals with AMD at elevated or reduced levels compared to healthy individuals. Exemplary AMD-associated proteins present at elevated levels in individuals with AMD include gi|178779|gb|AAA51748.1 (Apolipoprotein A-IV), gi|3337390|gb|AAC27432.1 (Haptoglobin), gil|82440|gb|AAB59530.1 (Fibrinogen gamma prime chain), and gi|223002|prf∥0401173A (Fibrin beta). Other exemplary AMD-associated proteins present at elevated levels in individuals with AND include gi|4558178|pdb|1QAB|D (Retinol Binding Protein), P01011 (Alpha-1 antichymotrypsin), and NP_000925.1 (Alpha-2 antiplasmin). Exemplary AMD-associated proteins present at decreased levels in individuals with AMD include gi|7023232|dbj|BAA91891.1 (Unnamed protein product), gi|28373620|pdb|1MA9|A (Vitamin D binding protein), gi|5174525|ref|NP_005900.1 (Microtubule-associated protein 1B isoform 1), gi|8928566|sp|Q02952|AK12_ HUMAN (A-kinase anchor protein 12 (AKAP 250) (Myasthenia gravis autoantigen gravin)), gi|15099973|gb|AAK84185.1 (Ig heavy chain variable region (anti-thrombospondin)), gi|41406064|ref|NP_005955.1 (Myosin, heavy polypeptide 10, non-muscle), gi|17318569|ref|NP_906112.2 (Keratin 1), gi|86854|gb|AAA36153.1 (Keratin type II subunit protein), gi|1346640|sp|P35580|MYHA_HUMAN (Myosin heavy chain, nonmuscle type B), gi|22761659|db|BAC11646.1 (Unnamed protein product), gi|544379|sp|P35574|GDE_RABIT (Glycogen debranching enzyme (Glycogen debrancher)), gi|4838009|gb|AAD30796.1 (Ig heavy chain variable region), gi|1154664|emb|CAA62603.1 (Ataxia telangectasia mutated (A-T)), gi|34365215|emb|CAE45949.1 (TNN13-interacting kinase (FPGT-encoded)), gi|339685|gb|AAA61181.1 (Transthyretin), gi|31615331|pdb|1HK3|A (Serum Albumin (mutant R218p)), gi|18044197|gb|AAH20197.1 (1-aminocyclopropanc-1-carboxylate synthase), gi|546095|gb|AAB30327.1 (Ig heavy chain variable region (anti-bistone H1 WRI-170 antibody)), gi|51476396|emb|CAH18188.1 (Alpha-2 macroglobulin), P06727 (Apolipoprotein A-IV), gi|51467959|ref|XP_497224.1 (BMS1-like (similar to KIAA0187; ribosomal)), gi|23273927|gb|AAH35014.1 (NudC domain containing 3 (KIAA1068 protein)), gi|34526199|dbj|BAC85198.1 (Unnamed protein product), P25311 (Zinc alpha-2 glycoprotein), gi|28466999|ref|NP_787057.1 (ARG99 protein), gi|404108|dbj|BAA03864.1 (Glutathione peroxidase), gi|50255295|gb|EAL18030.1 (Hypothetical protein CNBK0510), gi|7428606|pir|MHHUM (Ig mu chain C region, membrane-bound splice form), gi|38649048|gb|AAH62986.1 (ZFR protein), gi|37790798|gb|AAR03501.1 (Angiotensinogen, member 8), gi|24308400|ref|NT_612366.1 (Chromosome 10 open reading frame 42), gi|14625439|dbj|BAB61902.1 (KIAA1774 protein), gi|47124562|gb|AAH70299.1 (Haptoglobin), gi|539627|pir∥A46546 (Leukocyte common antigen long splice form), gi|4557225|ref|NP_000005.1 (Alpha-2 macroglobulin), gi|14600217|gb|AAK71298.1 (TCR beta chain Vbeta13S3), gi|114006|sp|P06727|APA4_HUMAN (Apolipoprotein A-IV), gi|40788364|dbj|BAA34505.2 (KIAA0785 protein), gi|7023207|dbj|BAA91880.1 (BTB and kelch domain containing 4 protein), gi|862457|dbj|BAA03941.1 (Enoyl-CoA hydratase/3-hydroxyacyl-CoA dehydrogenase alpha-subunit of trifunctional protein), gi|7022921|dbj|BAA91769.1 (LEPRFL1 protein), gi|10437767|dbj|BAB15104.1 (Zinc finger protein 2), gi|21071030|ref|NP_570602.2 (Alpha 1B glycoprotein), gi|28207863|emb|CAD62585.1 (Alpha-1 antitrypsin), gi|51471047|ref|XP_370690.3 (AT rich interactive domain 2 (ARID, RFX-like)), gi|6470150|gb|AAF13605.1 (BiP protein), gi|11138513|gb|AAG31421.1 (FcRn alpha chain), gi|10120958|pdb|1EXU|A (MHC-related Fc Receptor), gi|37747855|gb|AAH59367.1 (Transferrin), gi|339486|gb|AAA61148.1 (Transferrin), gi|5817245|emb|CAB53743.1 (dJ20N2.2 (novel protein similar to tubulin, beta polypeptide 4, member Q (TUBB4Q)), gi|51476755|emb|CAH18343.1 (Hypothetical protein), gi|50363219|ref|NP_001002236.1 (Alpha-1 antitrypsin, member 1), gi|28566306|gb|AA043053.1 (Heat shock regulate-1), gi|7428607|pir∥MHHU (Ig mu chain C region, secreted splice form), gi|1703025|sp|P01009 (Alpha-1 antitrypsin), gi|72059|pir∥NBHUA2 (Leucme-rich alpha-2-glycoprotein), gi|33469917|ref|NP_877423.1 (Minichromosome maintenance protein 4), gi|6650772|gb|AAF22007.1 (Serotransferrin), gi|553788|gb|AAA61141.1 (Transferrin), and gi|47077177|dbj|BAD18510.1 (ZNF438 variant 3). Other exemplary AMD-associated proteins differentially present in individuals with AMD include complement related proteins, including gi|40786791|gb|AAR89906.1 (Complement component 3), gi|22218654|pdb|1GPZ|B (Complement component 1, r subcomponent), and gi|39573703|dbj|BAC19850.2 (complement component 1, r subcomponent).

The invention provides methods for diagnosing AMD, assessing the efficacy of treatment of AMD, and monitoring the progression of AMD by determining the levels of one or more biomarkers in an individual and comparing the levels of the one or more biomarkers to an earlier determined levels or reference levels of the one or more biomarkers. Determination that a biomarker is at a level characteristic of a disease state in a subject suggests that the tested subject has or may be developing the disease, while determination that a biomarker is at a level characteristic of a non-disease state in a subject suggests that the tested subject does not have or is not developing the disease. Likewise, a change of biomarker levels over time to a level closer to that of a disease state suggests progression of the disease, while change of biomarker levels over time to a level closer to that of a non-disease state suggests regression of the disease (*e*.*g*., therapeutic efficacy).

In one embodiment, the methods for diagnosing AMD, assessing the efficacy of treatment of AMD, or monitoring the progression of AMD involves determining the levels of at least two biomarkers in an individual and comparing the levels of the at least two biomarkers to earlier determined levels and/or to reference levels of the at least two biomarkers.

In one embodiment, at least one of the at least two biomarkers for diagnosing AMD, assessing the efficacy of treatment of AMD, or monitoring the progression of AMD in an individual are complement related proteins indicated in Tables 2, 4, 5 or 6.

In one embodiment, at least one of the at least two biomarker for diagnosing AMD, assessing the efficacy of treatment of AMD, or monitoring the progression of AMD in an individual are immune related proteins indicated in Tables 2, 4, 5 or 6.

In one embodiment, at least one of the at least two biomarkers for diagnosing AMD, assessing the efficacy of treatment of AMD, or monitoring the progression of AMD in an individual are structural proteins indicated in Tables 2, 4, 5 or 6.

In one embodiment, at least one of the at least two biomarkers for diagnosing AMD, assessing the efficacy of treatment of AMD, or monitoring the progression of AMD in an individual are enzymes indicated in Tables 2, 4, 5 or 6.

In one embodiment, at least one of the at least two biomarkers for diagnosing AMD, assessing the efficacy of treatment of AMD, or monitoring the progression of AMD are proteins of unknown or undetermined function in Tables 2, 4, 5 or 6.

In a first aspect, the invention provides a method for diagnosing AMD in an individual, by determining the levels of one or more biomarkers in a sample from the individual, and comparing the levels of the one or more biomarkers in the sample from the individual to reference levels of the one or more biomarkers characteristic of a control population, where a difference in the levels of the one or more biomarkers between the sample from the individual and the control population indicates that the individual is developing or has AMD. The methods may include the steps of obtaining a sample from the individual and determining the levels of the one or more biomarkers. The levels of certain biomarkers are significantly different in individuals with AMD than in healthy individuals. The levels of certain biomarkers are higher in individuals with AMD than in healthy individuals. The levels of certain biomarkers are lower in individuals with AMD than in healthy individuals.

The levels of the one or more biomarkers can be determined by any suitable method, such as conventional techniques known in the art, including, for example and not for limitation, separation-based methods (*e*.*g*., gel electrophoresis), protein-based methods (*e*.*g*., mass spectroscopy), immunoassay methods (*e*.*g*., antibody-based detection), and function-based methods (*e*.*g*., enzymatic or binding activity).

In one embodiment, a method for diagnosing AMD in an individual involves obtaining a sample from the individual and determining the levels of the one or more biomarkers by separating proteins by 2-dimensional difference gel electrophoresis (DIGE).

The one or more biomarkers can be obtained in a biological sample, preferably a fluid sample, of the individual. The biological sample can also be a tissue sample, *e*.*g*., a skin biopsy. The precise sample to be taken from an individual may vary, but the sampling is typically minimally invasive and is easily performed by conventional techniques known in the art. The one or more biomarkers are preferentially obtained in a sample of the individual's blood, serum, plasma, urine, cerebral spinal fluid (CSF), or saliva.

In another aspect, the invention provides a method for assessing the efficacy of treatment of AMD in an individual, by determining the levels of one or more biomarkers in a sample from the individual before treatment or at a first time point after treatment, determining the levels of the one or more biomarkers in the individual at a later time point during treatment or after treatment, and comparing the levels of the one or more biomarkers at the two time points, where a difference in the levels of the one or more biomarkers between the two determinations in which the levels of the one or more biomarkers move closer to reference levels of the one or more biomarkers characteristic of a control population indicates that the treatment is effective. The methods may include the steps of obtaining a sample from the individual and determining the levels of the one or more biomarkers as above. The levels of certain biomarkers are higher in individuals with AMD than in healthy individuals. The levels of these biomarkers in individuals with AMD decreases (*i*.*e*., moves to a more normal level) upon treatment with an agent effective to treat AMD. The levels of certain other biomarkers are lower in individuals with AMD than in healthy individuals. The levels of these biomarkers in individuals with AMD increases (*i*.*e*., moves to a more normal level) upon treatment with an agent effective to treat AMD.

In one embodiment, a method for assessing the efficacy of treatment of AMD in an individual involves the individual being treated with an agent effective to treat the disease.

In one embodiment, a method for assessing the efficacy of treatment of AMD in an individual involves obtaining a sample from the individual and determining the levels of the one or more biomarkers by separating proteins by 2-dimensional difference gel electrophoresis (DIGE).

In one embodiment, a method for assessing the efficacy of treatment of AMD in a individual involves obtaining a sample of blood, serum, plasma or urine from the individual and determining the level of the one or more biomarkers.

For example, the invention provides a method for assessing the efficacy of treatment of AMD in an individual, by determining the levels of one or more biomarkers in a sample from the individual at a first time point during the course of treatment with an agent, determining the levels of the one or more biomarkers in the individual at a later time point during or after treatment with the agent, and comparing the levels of the one or more biomarkers at the two time points, where detection of more normal levels at a later time-point indicates regression of disease (*e*.*g*., therapeutic efficacy) and detection of levels less like the normal level at a later time-point indicates progression of disease. The methods may include the steps of obtaining a sample from the individual and determining the levels of the one or more biomarkers as above.

In another aspect, the invention provides a method for assessing the efficacy of treatment of AMD in an individual, comprising comparing levels of one or more biomarkers in a sample from the individual after administration of an agent to levels of the one or more biomarkers in a sample from the individual at an earlier time point and to reference levels of the one or more biomarkers characteristic of a control population, where a reduced difference between the levels of the one or more biomarkers in the individual after administration of the agent compared to the reference levels and the levels of the one or more biomarkers in the individual taken at an earlier time point compared to the reference levels in which the levels of the one or more biomarkers move closer to reference levels of the one or more biomarkers characteristic of a control population indicates that the treatment is effective. The methods may include the steps of obtaining a sample from the individual and determining the levels of the one or more biomarkers as above.

In another aspect, the invention provides a method for monitoring the progression of AMD, comprising detecting the levels of one or more biomarkers in a sample from the individual. In one embodiment, the individual is being administered with an agent effective to treat or prevent AMD, and the levels of the one or more biomarkers determines the future treatment regime for the individual. The methods may include the steps of obtaining a sample from the individual and determining the levels of the one or more biomarkers as above.

### BRIEF DESCRIPTION OF THE DRAWINGS

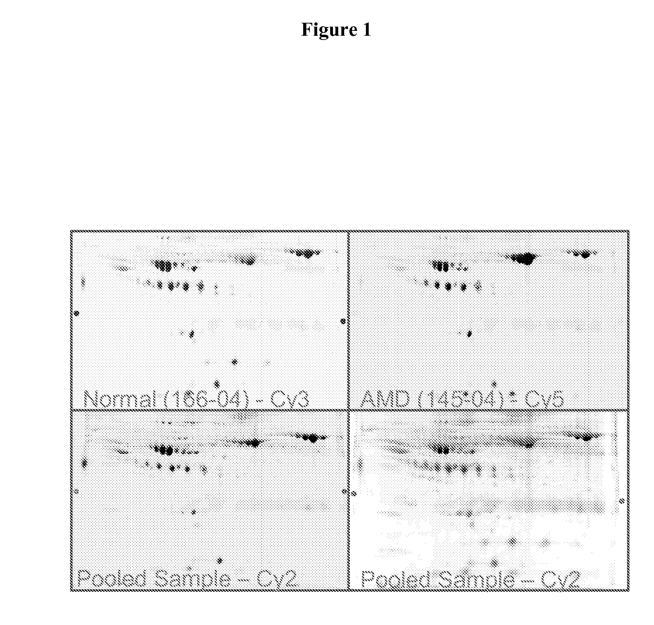

Figure 1. The DIGE system produces data for three samples in one gel, using different wavelengths of light to fluoresce the control (Normal (166-04) - Cy3), AMD (AMD (145-04) - Cy5) and pooled samples (Pooled Sample - Cy2).

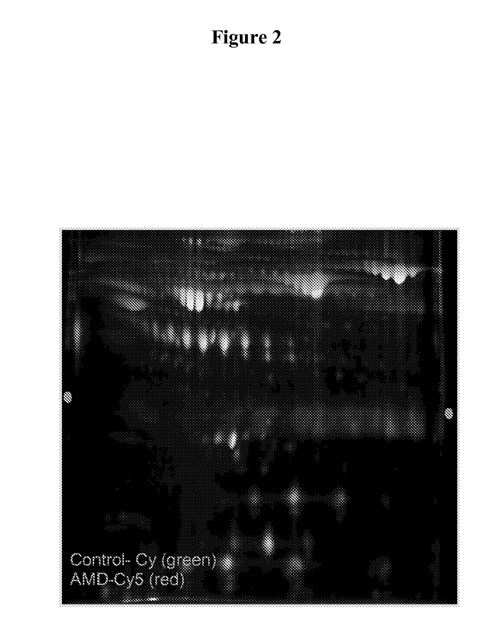

Figure 2. Merged 2D-DIGE overlay. Solid green or red spots represent proteins unique to control and AMD samples, respectively. Yellow spots represent proteins that are present in both samples, but not necessarily of equal proportion.

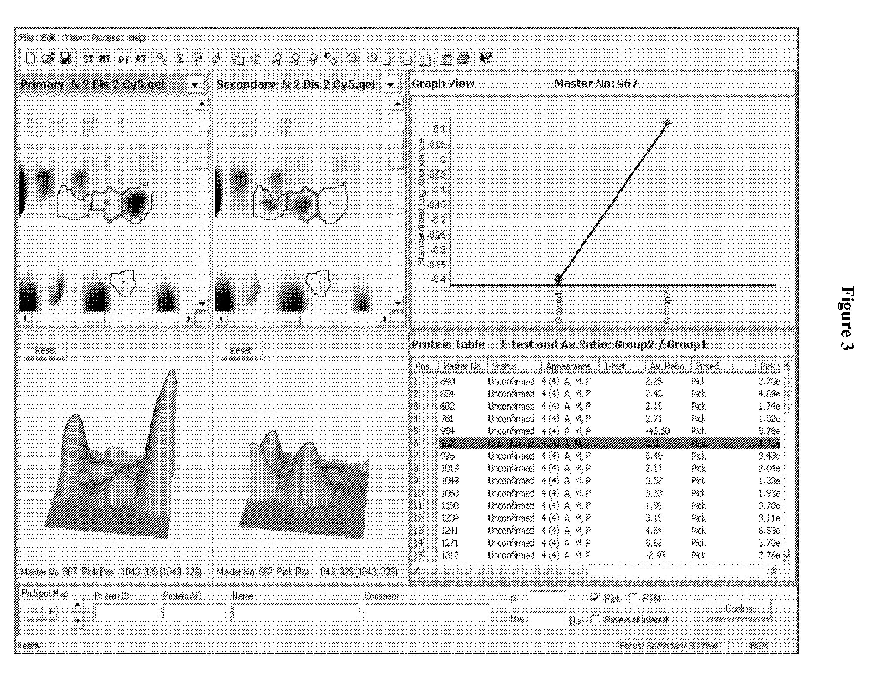

Figure 3. Representation of the analysis output of DeCyder software applied to the proteins separated by DIGE in Figure 2. The software matches spots across all gels, measures spot areas and volumes, and calculates fluorescent intensity group differences. This is most apparent in the graph view panel (above right), which depicts the mean intensities of the 2 groups for the selected gel spot.

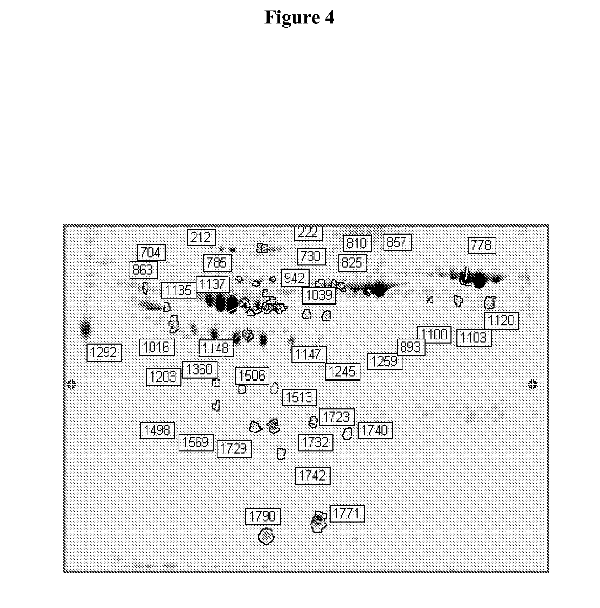

Figure 4. Image of the serum proteins from control individuals and AMD patients separated by DIGE in Figure 2 depicting the spots/proteins picked using an Ettan Spot Picker with >2-fold changes in fluorescent intensities. Each spot/protein that was picked for quantification was given a master spot number as indicated. The identity of the proteins in the spots, as determined by MALDI-ToF peptide mass fingerprinting analysis, is shown in Table 1.

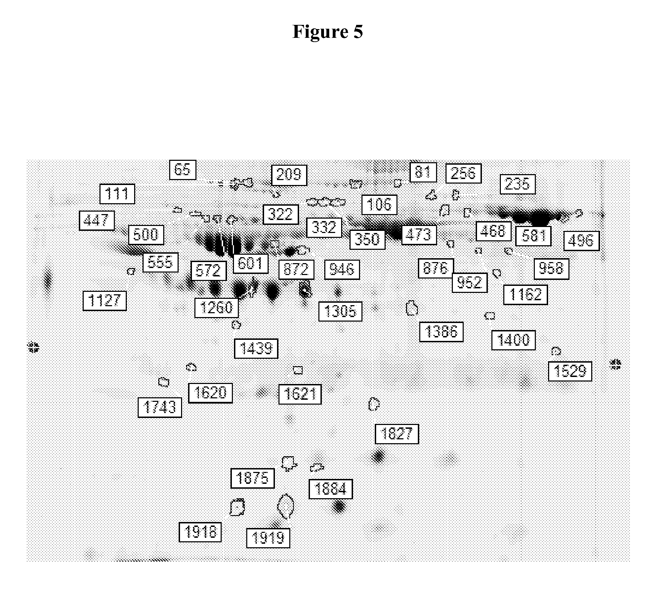

Figure 5. Image of the serum proteins from control individuals and AMD patients depicting the spots/proteins picked using an Ettan Spot Picker. Each spot/protein that was picked for quantification was given a master spot number as indicated. The identity of proteins corresponding to each master spot number is shown in Table 2.

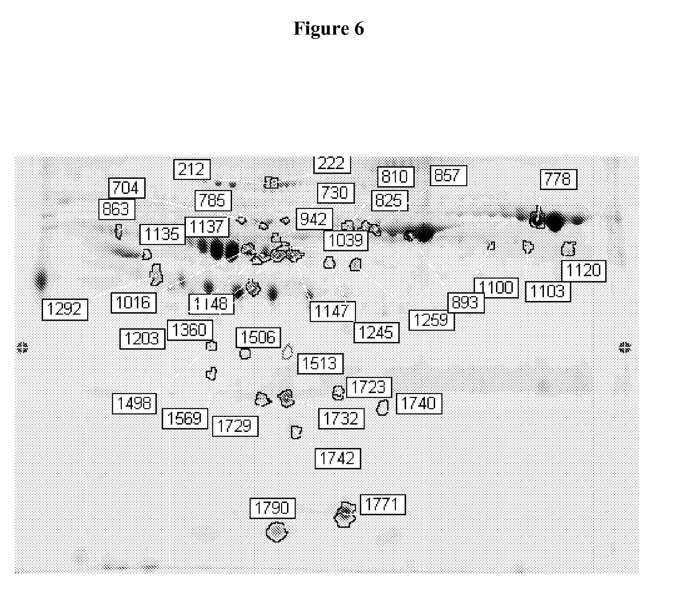

Figure 6. Image of the serum proteins from control individuals and AMD patients depicting the spots/proteins picked using an Ettan Spot Picker. Each spot/protein that was picked for quantification was given a master spot number as indicated. The identity of proteins corresponding to each master spot number is shown in Table 3.

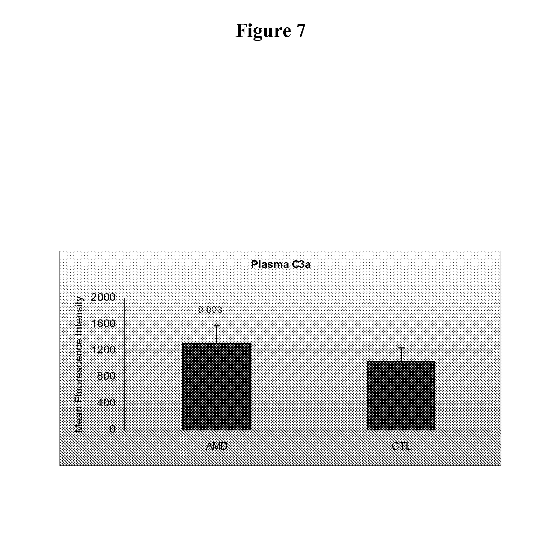

Figure 7. Mean fluorescence intensities of plasma C3a in AMD patients and control individuals. AMD plasma showed significantly higher levels (p<0.003) of C3a.

### DETAILED DESCRIPTION OF THE INVENTION

The invention relates to biomarkers associated with age-related macular degeneration (AMD). The biomarkers are proteins, the levels of which differ between individuals with AMD and age-matched control individuals. Certain of these biomarkers are present at elevated levels in individuals with AMD compared to controls. Certain other of these biomarkers are present at reduced levels in individuals with AMD compared to controls.

The invention provides methods for diagnosing AMD by determining the levels of one or more biomarkers in an individual and comparing the levels of the one or more biomarkers with reference levels characteristic of a control, healthy population. In one aspect, the invention provides methods for monitoring the progression of AND by determining the levels of one or more biomarkers in an individual with AMD being treated for the disease and comparing the levels of the one or more biomarkers to an earlier determined levels or reference levels of the biomarker. In one aspect, the invention provides methods for assessing the efficacy of treatment of AMD by determining the levels of one or more biomarkers in an individual with AMD being treated for the disease and comparing the levels of the one or more biomarkers to earlier determined levels or reference levels of the biomarker.

### I. DEFINITIONS

The following definitions are provided to aid in understanding the invention. Unless otherwise defined, all terms of art, notations and other scientific or medical terms or terminology used herein are intended to have the meanings commonly understood by those of skill in the arts of medicine and molecular biology. In some cases, terms with commonly understood meanings are defined herein for clarity and/or for ready reference, and the inclusion of such definitions herein should not be assumed to represent a substantial difference over what is generally understood in the art.

The term "biomarker" refers to a protein found at different levels in a biological fluid sample from an individual with AMD compared to an age-matched control individual.

The terms "complement protein" and "complement related protein" refer to any of more than 35 plasma or cell membrane proteins that make up the complement system, a biochemical cascade of the immune system that helps clear the body of pathogens. Complement related proteins as used herein refer to proteins that are involved in the classical complement pathway, the alternative complement pathway or the mannose-binding lectin pathway, and include, for example and not for limitation, activators C6, C7, C9, MBL2, and PFC, complexes C1Q (C1QA, C1QB, C1QG), C3-convertase, C8 (C8A, C8B, C8G), and membrane attack complex (MAC), enzymes BF, C1R, C1S, C2, C3, C4, C5, DF, MASP1, and MASP2, inhibitors Clinh, C4BP (C4BPA, C4BPB), CLU, DAF, complement factor H (CFH or HF1), SERPING1, and VTN, and receptors C3AR1, C5R1, CR1, CR2, and ITGAM.

The term "treating" or "treatment" refers to the treatment of a disease or condition in a mammal, preferably a human, in which the disease or condition has been diagnosed as AMD involving impairment of visual acuity. Treating or treatment includes inhibiting the disease or condition (*i*.*e*., arresting progression), relieving or ameliorating the disease or condition (*i*.*e*., causing regression), or preventing progression of the disease or condition (*i*.*e*., delaying progression). Treating or treatment can involve a course of treatment in which an individual with AMD is administered an agent more than once periodically over time that is expected to be effective in inhibiting, relieving or ameliorating, or preventing progression of the disease.

The term "agent" refers to a drug or drug candidate. An agent may be a naturally occurring molecule or may be a synthetic compound, including, for example and not for limitation, a small molecule (*e*.*g*., a molecule having a molecular wight < 1000), a peptide, a protein, an antibody, or a nucleic acid, used to treat an individual with AMD or other disease of the eye.

The term "level" refers to the amount of a biomarker in a biological sample obtained from an individual. The amount of the biomarker can be determined by any method known in the art and will depend in part on the nature of the biomarker (*e*.*g*., electrophoresis, including capillary electrophoresis, 1 - and 2-dimensional electrophoresis, 2-dimensional difference gel electrophoresis DIGE followed by MALDI-ToF mass spectroscopy, chromatographic methods such as high performance liquid chromatography (HPLC), than layer chromatography (TLC), hyperdiffusion chromatography, mass spectrometry (MS), various immunological methods such as fluid or gel precipitin reactions, single or double immunodiffusion, immunoelectrophoresis, radioimmunoassay (RIA), enzyme-linked immunosorbant assays (ELISA), immunofluorescent assays, Western blotting and others, and enzyme- or function-based activity assays). It is understood that the amount of the biomarker need not be determined in absolute terms, but can be determined in relative terms. For example, the amount of the biomarker may be expressed by its concentration in a biological sample, by the concentration of an antibody that binds to the biomarker, or by the functional activity (*i*.*e*., binding or enzymatic activity) of the biomarker.

The level(s) of a biomarker(s) can be determined as described above for a single biomarker or for a "set" of biomarkers. A set of biomarkers refers to a group of more than one biomarkers that have been grouped together, for example and not for limitation, by a shared property such as their presence at elevated levels in AMD patients compared to controls, by their presence at reduced levels in AMD patients compared to controls, by their ratio or difference in levels between AMD patients and controls (*e*.*g*., difference between 1.25- and 2-fold, difference between 2- and 3-fold, difference between 3- and 5-fold, and difference of at least 5-fold), or by function.

The term "difference" as it relates to the level of a biomarker of the invention refers to a difference that is statistically different. A difference is statistically different, for example and not for limitation, if the expectation is < 0.05, the p value determined using the Student's t-test is < 0.05, or if the p value determined using the Student's t-test is < 0.1. The difference in level of a biomarker between an individual with AMD and a control individual or population can be, for example and not for limitation, at least 10% different (1.10 fold), at least 25% different (1.25-fold), at least 50% different (1.5-fold), at least 100% different (2-fold), at least 200% different (3-fold), at least 400% different (5-fold), at least 10-fold different, at least 20-fold different, at least 50-fold different, at least 100-fold different, at least 150-fold, or at least 200-fold different.

The term "progression" refers to an increase in symptoms of AMD, including, for example and not for limitation, decreased visual acuity of an individual with AMD undergoing treatment for the disease.

The term "reference" as it relates to a biomarker of the invention refers to an amount of a biomarker in a healthy individual or control population. The reference level or amount may be determined by obtaining a sample and detecting the biomarker in a healthy individual, or may be determined by taking the level or amount known or readily determine from a control population.

The term "control" refers to an individual who has not been diagnosed as having AMD, or who has not displayed upon examination any symptoms characteristic of AMD, or a group of such individuals.

### II. BIOMARKERS

Biological compounds present in the blood, plasma, serum or other body fluid, or in a tissue sample, may be present at different levels in individuals with a disease or condition as compared to otherwise healthy individuals or a control population. The inventor has discovered that levels of particular serum proteins differ between individuals with AMD and age-matched control individuals.

In one aspect, the invention relates to biological compounds, in particular, proteins, that are differentially present in serum from individuals with AMD as compared to age-matched control individuals (individuals without the disease). These proteins are therefore associated with AMD and termed AMD-associated proteins (biomarkers). These biomarkers are present at different levels in individuals with AMD as compared to individuals without the disease. These biomarkers are present in individuals with AMD at either elevated or reduced levels compared to healthy individuals. Exemplary biomarkers shown to be present in individuals with AMD at different levels compared to age-matched control individuals are provided in Tables 1 to 7 and in Examples 1 to 3.

As discussed in the examples, biomarkers were identified by first separating proteins in a serum sample by 2-dimensional difference gel electrophoresis (DIGE), followed by identifying the proteins by MALDI-ToF mass spectroscopy. Figures 1 to 4 discussed in Example 1 show that DIGE, in combination with fluorescent dyes, was able to detect and to subsequently quantify the levels of thousands of spots (*i*.*e*., proteins). MALDI-ToF mass spectroscopy was then used to determine the identity of spots (*i*.*e.*, proteins) that were differentially present between individuals with AMD and control individuals. Table 1 in Example 1 lists 36 proteins that were identified via MALDI-TOF peptide mass fingerprinting analysis and shown to be present at significantly different levels in serum samples from individuals with AMD compared to controls.

In the practice of the invention biomarkers can be obtained in a biological sample, preferably a fluid sample, of the individual. The biomarkers are preferentially obtained in a sample of the individual's blood, serum, plasma, urine, CSF or saliva. The biological sample can also be a tissue sample, *e*.*g*., a skin biopsy.

In one embodiment, a method for assessing the efficacy of treatment of AMD in an individual involves obtaining a sample of blood, serum or plasma from the individual and determining the levels of one or more biomarkers. As an example, the biomarkers of the invention were obtained from the serum of individuals with AMD and age-matched control individuals, as described in Materials and Methods in Example 1.

### III. DETECTION OF BIOMARKERS ASSOCIATED WITH AMD

AMD biomarkers can be detected in any of a number of methods including immunological assays (*e*.*g*., ELISA), separation-based methods (*e*.*g*., gel electrophoresis), protein-based methods (*e*.*g*., mass spectroscopy), function-based methods (*e*.*g*., enzymatic or binding activity), or the like. Other methods will be known to those of skill in the art guided by this specification. The particular preferred method for determining the levels will depend, in part on the identity and nature of the biomarker protein.

In one embodiment, the method for separating and determining the levels of the one or more biomarkers of the invention involve obtaining a biological sample from a individual, separating and determining the levels of the proteins by 2-dimensional difference gel electrophoresis (DIGE), and identifying the proteins by MALDI-ToF mass spectroscopy, as shown in Example 1. In a related embodiment, the proteins separated by DIGE can be identified by comparison to a known separation pattern of proteins using DIGE.

In some cases it will be desirable to establish normal or baseline values (or ranges) for biomarker expression levels. Normal levels can be determined for any particular population, subpopulation, or group of organisms according to standard methods well known to those of skill in the art. Generally, baseline (normal) levels of biomarkers are determined by quantifying the amount of biomarker in biological samples (*e*.*g*., fluids, cells or tissues) obtained from normal (healthy) subjects. Application of standard statistical methods used in medicine permits determination of baseline levels of expression, as well as significant deviations from such baseline levels.

In carrying out the diagnostic and prognostic methods of the invention, as described above, it will sometimes be useful to refer to "diagnostic" and "prognostic values." As used herein, "diagnostic value" refers to a value that is determined for the biomarker gene product detected in a sample which, when compared to a normal (or "baseline") range of the biomarker gene product is indicative of the presence of a disease. "Prognostic value" refers to an amount of the biomarker that is consistent with a particular diagnosis and prognosis for the disease. The amount of the biomarker gene product detected in a sample is compared to the prognostic value for the cell such that the relative comparison of the values indicates the presence of disease or the likely outcome of the disease progression. In one embodiment, for example, to assess AMD prognosis, data are collected to obtain a statistically significant correlation of biomarker levels with different AMD classes or grades. A predetermined range of biomarker levels is established from subjects having known clinical outcomes. A sufficient number of measurements is made to produce a statistically significant value (or range of values) to which a comparison will be made.

It will be appreciated that the assay methods do not necessarily require measurement of absolute values of biomarker, unless it is so desired, because relative values are sufficient for many applications of the methods of the present invention. Where quantification is desirable, the present invention provides reagents such that virtually any known method for quantifying gene products can be used.

### IV. DIAGNOSIS OF AMD

In a first aspect, the invention provides a method for diagnosing AMD in a individual, by determining the levels of one or more biomarkers in a sample from the individual, and comparing the levels of the one or more biomarkers in the sample from the individual to reference levels of the biomarkers characteristic of a control population, where a difference in the levels of the one or more biomarkers between the sample from the individual and the control population indicates that the individual has AMD. The methods include obtaining a sample from the individual and determining the levels of the one or more biomarkers. The levels of certain biomarkers are significantly different in individuals with AMD than in healthy individuals. The levels of certain biomarkers are higher in individuals with AMD than in healthy individuals. The levels of certain biomarkers are lower in individuals with AMD than in healthy individuals. The biomarkers can be obtained in a biological sample, and the levels of the one or more biomarkers can be determined, by any suitable method, as described above.

As used herein, the term "diagnosis" is not limited to a definitive or near definitive determination that an individual has a disease, but also includes determining that an individual has an increased likelihood of having or developing the disease, compared to healthy individuals or to the general population.

In one embodiment, a method for diagnosing AMD in a individual involves obtaining a sample from the individual and determining the levels of the one or more biomarkers by separating proteins by 2-dimensional difference gel electrophoresis (DIGE) or other methods.

In one embodiment, a method for diagnosing AMD in a individual involves obtaining a sample of blood, serum, plasma or urine from the individual and determining the levels of the one or more biomarkers.

In one embodiment, the method for diagnosing AMD involves determining the levels of one biomarker. An example of a single biomarker that may be used is the complement activation byproduct C3a, as shown in Figure 7 in Example 3.

In one embodiment, the method for diagnosing AMD involves determining the levels of a set of biomarkers (*i*.*e*., more than one biomarker). The biomarkers in a particular set may be related or grouped in a number of ways. By measuring multiple biomarkers, conclusions can be reached that are more precise and with higher confidence. The biomarkers in a set may be related by their function, for example, proteins associated with immune-mediated and inflammatory pathways, immunoglobulins, serum enzymes, and structural proteins. An example of such a set of biomarkers is provided in Table 7, below. The biomarkers in a set may be related by the magnitude of the difference in their levels between individuals with AMD and control individuals. For example, in one embodiment the levels of biomarkers in controls compared to AMD patients differs by a factor of at least 1.5-fold, sometime at least 2-fold and sometimes at least 2.5-fold. Other sets include biomarkers having an at least 1.25-fold, at least 3 -fold, at least 4-fold, at least 5-fold, or at least 10-fold difference between AMD patients and control individuals. In one embodiment, biomarkers in a set are related by the direction of change in AMD patients compared to controls, *i*.*e*., at elevated (see Tables 4 and 5) or reduced (see Table 6) levels.

In one embodiment, the method for diagnosing AMD involves determining the levels of a set of biomarkers (*i*.*e*., more than one biomarker) in which all of the biomarkers in the set are present at elevated levels in individuals with AMD as compared to control individuals. Examples of such a set of biomarkers are provided in Tables 4 and 5 below. In one embodiment, the set of biomarkers can comprise at least 2, at least 3, or at least 4of the biomarkers listed in Table 4. In one embodiment, the set of biomarkers can comprise at least 2, or at least 3 of the biomarkers listed in Table 5. In one embodiment, the set of biomarkers can comprise at least 2, at least 3, at least 4, or at least 5 of the biomarkers listed in Tables 4 and 5, taken together.

In one embodiment, the method for diagnosing AMD involves determining the levels of a set of biomarkers (*i*.*e*., more than one biomarker) in which all of the biomarkers in the set are present at reduced levels in individuals with AMD as compared to control individuals. An example of such a set of biomarkers is provided in Table 6. In one embodiment, the set of biomarkers can comprise at least 2, at least 3, at least 4, or at least 5 of the biomarkers listed in Table 6.

In one embodiment, the method for diagnosing AMD involves determining the levels of a set of biomarkers such as, without limitation, those sets described in Section VII below.

### V. BIOMARKERS AS SURROGATE ENDPOINTS FOR ASSESSING THE EFFICACY OF TREATMENT OF AMD

In a first aspect, the invention provides a method for assessing the efficacy of treatment of AMD in an individual, comprising determining the levels of one or more biomarkers in a sample from the individual before treatment or at a first time point after treatment, and determining the levels of the one or more biomarkers in the individual at a later time point or time points during treatment or after treatment, and comparing the levels of the one or more biomarkers at the two or more time points. A change from a level characteristic of AMD to a more normal level is an indication of efficacy of the treatment. The methods include obtaining a sample from the individual and determining the levels of the one or more biomarkers. The levels of certain biomarkers are higher in individuals with AMD than in healthy individuals. The levels of these biomarkers in an individual with AMD decrease upon treatment with an agent effective to treat AMD. The levels of certain other biomarkers are lower in individuals with AMD than in healthy individuals. The levels of these biomarkers in an individual with AMD increase upon treatment with an agent effective to treat AMD. The methods include obtaining a sample from the individual and determining the levels of the one or more biomarkers as above.

In one embodiment, a method for assessing the efficacy of treatment of AMD in an individual involves the individual being treated with an agent effective to treat the disease.

In one embodiment, a method for assessing the efficacy of treatment of AMD in an individual involves obtaining a sample from the individual and determining the levels of the one or more biomarkers by separating proteins by 2-dimensional difference gel electrophoresis (DIGE) or other methods.

In one embodiment, a method for assessing the efficacy of treatment of AMD in an individual involves obtaining a sample of blood, serum, plasma or urine from the individual and determining the levels of the one or more biomarkers.

In one embodiment, the method for assessing the efficacy of treatment of AMD involves determining the levels of one biomarker. An example of a single biomarker that may be used is the complement activation by-product C3a, as shown in Figure 7.

In one embodiment, the method for assessing the efficacy of treatment of AMD involves determining the levels of a set of biomarkers (*i*.*e*., more than one biomarker). Sets may be defined, for example, as described above.

In a second aspect, the invention provides a method for assessing the efficacy of treatment of AMD in an individual, comprising first determining the levels of one or more biomarkers in a sample from the individual at a first time point during the course of treatment with an agent, and determining the levels of the one or more biomarkers in a sample from the individual at multiple later time points during or after treatment with the agent, and second comparing the levels of the one or more biomarkers at the first time point and the later time point. The methods include obtaining a sample from the individual and determining the levels of the one or more biomarkers as above.

In a third aspect, the invention provides a method for assessing the efficacy of treatment of AMD in an individual, comprising comparing the levels of one or more biomarkers in a sample from the individual after administration of an agent to the levels of the one or more biomarkers in a sample from the same individual taken at an earlier time point and to reference levels of the one or more biomarkers characteristic of a control population, wherein a reduced difference between the levels of the one or more biomarkers in the individual after administration of the agent compared to the reference levels and the levels of the one or more biomarkers in the individual taken at an earlier time point compared to the reference levels indicates that the treatment is effective. Methods to determine the reference levels of the one or more biomarkers characteristic of a control population are well-known in the art. The methods can include obtaining a sample from the individual and determining the levels of the one or more biomarkers as above.

In one embodiment, the method for assessing the efficacy of treatment of AMD involves determining the levels of a set of biomarkers such as, without limitation, those sets described in Section VII below.

### VI. MONITORING PROGRESSION OF AMD

In one aspect, the invention provides a method for monitoring the progression of AMD, comprising detecting one of more biomarkers in a sample from the individual. In one embodiment, the individual is under treatment with an agent effective to treat or prevent AMD, and the levels of the one or more biomarkers determines the future treatment regime for the individual. The methods include obtaining a sample from the individual and determining the levels of the one or more biomarkers as above.

In one embodiment, a method for monitoring the progression of treatment of AMD in an individual involves obtaining a sample from the individual and determining the levels of the one or more biomarkers by separating proteins by 2-dimensional difference gel electrophoresis (DICE) or other methods.

In one embodiment, a method for monitoring the progression of treatment of AMD in an individual involves obtaining a sample of blood, serum, plasma or urine from the individual and determining the levels of the one or more biomarkers.

In one embodiment, the method for monitoring the progression of treatment of AMD involves determining the levels of one biomarker. An example of a single biomarker that may be used is the complement activation byproduct C3a, as shown in Figure 7.

In one embodiment, the method for monitoring the progression of treatment of AMD involves determining the levels of a set of biomarkers (*i*.*e*., more than one biomarker). The biomarkers in a set may be related by their function, for example, proteins associated with immune-mediated and inflammatory pathways. An example of such a set of biomarkers is provided in Table 7.

In one embodiment, the method for monitoring the progression of treatment of AMD involves determining the levels of a set of biomarkers such as, without limitation, those sets described in Section VII below.

### VII. EXEMPLARY SETS OF BIOMARKERS

In one aspect, the invention provides a method for diagnosing age-related macular degeneration (AMD) in an individual, by determining levels of at least one, preferably at least two, AMD-associated protein markers (biomarkers) in a sample from the individual, and comparing the levels of the biomarkers in the sample to reference levels of the biomarkers characteristic of a control population of individuals without AMD, where a difference in the levels of the biomarkers between the sample from the individual and the control population indicates that the individual has an increased likelihood of having AMD. In some embodiments, at least one of the biomarkers is other than a complement related protein. In some embodiments, at least one of the biomarkers is a complement related protein expressed at elevated levels in individuals with AMD. In some embodiments, at least one of the biomarkers is a complement related protein expressed at decreased levels in individuals with AMD. In some embodiments, at least one of the biomarkers is an immune related protein expressed at elevated levels in individuals with AMD. In some embodiments, at least one of the biomarkers is an immune related protein expressed at decreased levels in individuals with AMD. In some embodiments, at least one of the biomarkers is a structural protein expressed at elevated levels in individuals with AMD. In some embodiments, at least one of the biomarkers is a structural protein expressed at decreased levels in individuals with AMD. In some embodiments, at least one of the biomarkers is an enzyme expressed at elevated levels in individuals with AMD. In some embodiments, at least one of the biomarkers is an enzyme expressed at decreased levels in individuals with AMD. In some embodiments, the biomarker is not a complement related protein. In some embodiments, the biomarker is not an immune related protein. In some embodiments, the biomarker is not a structural protein. In some embodiments, the biomarker is not an enzyme.

In another aspect, the invention provides a method for assessing the efficacy of a treatment for age-related macular degeneration (AMD) in an individual, by (a) determining levels of at least one, preferably at least two, biomarkers in a sample from the individual either before treatment or at a first time point after treatment with an agent and (b) determining levels of the biomarkers in a sample from the individual at a later time point during treatment or after treatment with the agent, where a difference in the levels of the biomarkers measured in (b) compared to (a) in which the levels of the biomarkers moves closer to reference levels of the biomarkers characteristic of a control population of individuals without AMD indicates that the treatment is effective, In some embodiments, at least one of the biomarkers is other than a complement related protein. In some embodiments, at least one of the biomarkers is a complement related protein expressed at elevated levels in individuals with AMD. In some embodiments, at least one of the biomarkers is a complement related protein expressed at decreased levels in individuals with AMD. In some embodiments, at least one of the biomarkers is an immune related protein expressed at elevated levels in individuals with AMD. In some embodiments, at least one of the biomarker is an immune related protein expressed at decreased levels in individuals with AMD. In some embodiments, at least one of the biomarkers is a structural protein expressed at elevated levels in individuals with AMD. In some embodiments, at least one of the biomarkers is a structural protein expressed at decreased levels in individuals with AMD. In some embodiments, at least one of the biomarkers is an enzyme expressed at elevated levels in individuals with AMD. In some embodiments, at least one of the biomarkers is an enzyme expressed at decreased levels in individuals with AMD. In some embodiments, the biomarker is not a complement related protein. In some embodiments, the biomarker is not an immune related protein. In some embodiments, the biomarker is not a structural protein. In some embodiments, the biomarker is not an enzyme.

In one embodiment, the method for diagnosing AMD or assessing the efficacy of a treatment for AMD involves determining the levels of biomarkers where at least one of the biomarkers is a complement related protein expressed at elevated levels in individuals with AMD (*e*.*g*., as described in Tables 4 or 5).

In one embodiment, the method for diagnosing AMD or assessing the efficacy of a treatment for AMD involves determining the levels of biomarkers where at least one of the biomarkers is a complement related protein expressed at decreased levels in individuals with AMD (*e*.*g*., as described in Table 6).

In one embodiment, the method for diagnosing AMD or assessing the efficacy of a treatment for AMD involves determining the levels of biomarkers where at least one of the biomarkers is an immune related protein expressed at elevated levels in individuals with AMD (*e*.*g*., as described in Tables 4 or 5).

In one embodiment, the method for diagnosing AMD or assessing the efficacy of a treatment for AMD involves determining the levels of biomarkers where at least one of the biomarkers is an immune related protein expressed at decreased levels in individuals with AMD (*e*.*g*., as described in Table 6).

In one embodiment, the method for diagnosing AMD or assessing the efficacy of a treatment for AMD involves determining the levels of biomarkers where at least one of the biomarkers is a structural protein expressed at elevated levels in individuals with AMD (*e*.*g*., as described in Tables 4 or 5).

In one embodiment, the method for diagnosing AMD or assessing the efficacy of a treatment for AMD involves determining the levels of biomarkers where at least one of the biomarkers is a structural protein expressed at decreased levels in individuals with AMD (*e*.*g*., as described in Table 6).

In one embodiment, the method for diagnosing AMD or assessing the efficacy of a treatment for AMD involves determining the levels of biomarkers where at least one of the biomarkers is an enzyme expressed at elevated levels in individuals with AMD (*e*.*g*., as described in Tables 4 or 5).

In one embodiment, the method fordiagnosing AMD or assessing the efficacy of a treatment for AMD involves determining the levels of biomarkers where at least one of the biomarkers is an enzyme expressed at elevated levels in individuals with AMD (*e*.*g*., as described in Table 6).

In one embodiment, the methods involve determining the levels of bioanarkers that are immune related proteins selected from the group consisting of: gi|3337390|gb|AAC27432.1 (Haptoglobin), gi|182440|gb|AAB59530.1 (Fibrinogsn gamma prime chain), gi|223002|prf∥0401173A (Fibrin beta), gi|4558178|pdb|1QAB|D (Retinol Binding Protein), gi|28373620|pdb|1MA9|A (Vitamin D binding protein), gi|15099973|gb|AAK84185.1 (Ig heavy chain variable region (anti-thrombospondin)), gi|4838009jgbjAAD30796.1 (Ig heavy chain variable region), gi|546095|gb|AAB30327.1 (Ig heavy chain variable region (anti-histone H1 WRI-170 antibody)), gi|7428606|pir∥MHHUM (Ig mu chain C region, membrane-bound splice form), gi|47124562|gb|AAH70299.1 (Haptoglobin), gi|539627|pir∥A46546 (Leukocyte common antigen long splice form), gi|14600217|gb|AAK71298.1 (TCR beta chain Vbeta13S3), gi|11138513|gb|AAG31421.1 (FcRn alpha chain), gi|10120958|pdb|1EXU|A (MHC-related Fc Receptor), and gi|7428607|pir∥MHHU (Ig mu chain C region, secreted splice form).

In one embodiment, the methods involve determining the levels of biomarkers that are structural proteins selected from the group consisting of: gi|178779|gb|AAA51748.1 (Apolipoprotein A-IV), P01011 (Alpha-1 antichymotrypsin), NP_000925.1 (Alpha-2 antiplasmin), gi|5174525|ref|NP_005900.1 (Microtubule-associated protein 1B isoform 1), gi|8928566|sp|Q02952|AK12_HUMAN (A-kinase anchor protein 12 (AKAP 250) (Myasthenia gravis autoantigen gravin)), gi|41406064|ref|NP_005955.1 (Myosin, heavy polypeptide 10, non-muscle), gi|17318569|ref|NP_006112.2 (Keratin 1), gi|386854|gb|AAA36153.1 (Keratin type II subunit protein), gi|1346640|sp|P35580|MYHA_HUMAN (Myosin heavy chain, nonmuscle type B), gi|1154664|emb|CAA62603.1 (Ataxia telangectasia mutated (A-T)), gi|339685|gb|AAA61181.1 (Transthyretin), gi|31615331|pdb|1HK3|A (Serum Albumin (mutant R218p)), gi|51476396|emb|CAH18188.1 (Alpha-2 macroglobulin), P06727 (Apolipoprotein A-IV), gi|51467959|ref|XP_497224.1 (BMS1-like (similar to KIAA0187; ribosomal)), gi|23273927|gb|AAH35014.1 (NudC domain containing 3 (KIAA1068 protein)), P25311 (Zinc alpha-2 glycoprotein), gi|28466999|ref|NP_787057.1 (ARG99 protein), gi|38649048|gb|AAH62986.1 (ZFR protein), gi|37790798|gb|AAR03501.1 (Angiotensinogen, member 8), gi|4557225|ref|NP_000005.1 (Alpha-2 macroglobulin), gi|114006|sp|P06727|APA4_HUMAN (Apolipoprotein A-IV), gi|7023207|dbj|BAA91880.1 (BTB and kelch domain containing 4 protein), gi|7022921|dbj|BAA91769.1 (LEPREL1 protein), gi|10437767|dbj|BAB15104.1 (Zinc finger protein 2), gi|21071030|ref|NP_570602.2 (Alpha 1B glycoprotein), gi|28207863|emb|CAD62585.1 (Alpha-1 antitrypsin), gi|51471047|ref|XP_370690.3 (AT rich interactive domain 2 (ARID, RFX-like)), gi|6470150|gb|AAF13605.1 (BiP protein), gi|37747855|gb|AAH59367.1 (Transferrin), gi|339486|gb|AAA61148.1 (Transferrin), gi|5817245|emb|CAB53743.1 (dJ20N2.2 (novel protein similar to tubulin, beta polypeptide 4, member Q (TUBB4Q)), gi|50363219|ref|NP_001002236.1 (Alpha-1 antitrypsin, member 1), gi|28566306|gb|AA043053.1 (Heat shock regulate-1), gi|1703025|sp|P01009 (Alpha-1 antitrypsin), gi|72059|pir∥NBHUA2 (Leucine-rich alpha-2-glycoprotein), gi|33469917|ref|NP_877423.1 (Minichromosome maintenance protein 4), gi|6650772|gb|AAF22007.1 (Serotransferrin), gi|553788|gb|AAA61141.1 (Transferrin), and gi|47077177|dbj|BAD18510.1 (ZNF438 variant 3).

In one embodiment, the methods involve determining the levels of biomarkers that are enzymes selected from the group consisting of: gi|544379|sp|P35574|GDE_RABIT (Glycogen debranching enzyme (Glycogen debrancher)), gi|34365215|emb|CAE45949.1 (TNN13-interacting kinase (FPGT-encoded)), gi|18044197|gb|AAH20197.1 (1-aminocyclopropane-1-carboxylate synthase), gi|404108|dbj|BAA03864.1 (Glutathione peroxidase), and gi|862457|dbj|BAA03941.1 (Enoyl-CoA hydratase/3-hydroxyacyl-CoA dehydrogenase alpha-subunit of trifunctional protein).

In one embodiment, the methods involve determining the levels of biomarkers that are proteins selected from the group consisting of: gi|7023232|dbj|BAA91891.1 (Unnamed protein product), gi|22761659|dbj|BAC11646.1 (Unnamed protein product), gi|50255295|gb|EAL18030.1 gi|34526199|dbj|BAC85198.1 (Unnamed protein product), (Hypothetical protein CNBK0510), gi|24308400|ref|NP_612366.1 (Chromosome 10 open reading frame 42), gi|14625439|dbj|BAB61902.1 (KIAA1774 protein), gi|40788364|dbj|BAA34505.2 (KIAA0785 protein), and gi|51476755|emb|CAH18343.1 (Hypothetical protein).

In one embodiment, the methods involve determining the levels of biomarkers wherein at least one of the biomarkers is a complement related protein and at least one of the biomarkers is an immune related protein.

In one embodiment, the methods involve determining the levels of biomarkers wherein at least one of the biomarkers is a complement related protein and at least one of the biomarkers is a structural protein.

In one embodiment, the methods involve determining the levels of biomarkers wherein at least one of the biomarkers is a complement related protein and at least one of the biomarkers is an enzyme.

In one embodiment, the methods involve determining the levels of biomarkers wherein at least one of the biomarkers is an immune related protein and at least one of the biomarkers is a structural protein.

In one embodiment, the methods involve determining the levels of biomarkers wherein at least one of the biomarkers is an immune related protein and at least one of the biomarkers is an enzyme.

In one embodiment, the methods involve determining the levels of biomarkers wherein at least one of the biomarkers is a structural protein and at least one of the biomarkers is an enzyme.

In one embodiment, at least one, optionally both, of the at least two biomarkers for diagnosing AMD, assessing the efficacy of treatment of AMD, or monitoring the progression of AMD in an individual are not complement related proteins indicated in Tables 2, 4, 5 or 6.

In one embodiment, at least one, optionally both, of the at least two biomarkers for diagnosing AMD, assessing the efficacy of treatment of AMD, or monitoring the progression of AMD in an individual are not immune related proteins indicated in Tables 2, 4, 5 or 6.

In one embodiment, at least one, optionally both, of the at least two biomarkers for diagnosing AMD, assessing the efficacy of treatment of AMD, or monitoring the progression of AMD in an individual are not structural proteins indicated in Tables 2, 4, 5 or 6.

In one embodiment, at least one, optionally both, of the at least two biomarkers for diagnosing AMD, assessing the efficacy of treatment of AMD, or monitoring the progression of AMD in an individual are not enzymes indicated in Tables 2, 4, 5 or 6.

In one embodiment, at least one, optionally both, of the at least two biomarkers for diagnosing AMD, assessing the efficacy of treatment of AMD, or monitoring the progression of AMD are not proteins of unknown or undetermined function in Tables 2, 4, 5 or 6.

In one embodiment, the methods involve determining the levels of a set of biomarkers, wherein the set of biomarkers includes at least 2, at least 3, or at least 4 of the other biomarkers listed in Table 4.

In one embodiment, the methods involve determining the levels of a set of biomarkers, wherein the set of biomarkers includes at least 2, or at least 3 of the other biomarkers listed in Table 5.

In one embodiment, the methods involve determining the levels of a set of biomarkers, wherein the set of biomarkers includes at least 2, at least 3, at least 4, at least 5, at least 6, at least 7, at least 8, at least 9, at least 10, or at least 15 of the other biomarkers listed in Tables 4 and 6.

In one embodiment, the methods involve determining the levels of a set of biomarkers, wherein the set of biomarkers includes at least 2, at least 3, at least 4, at least 5, at least 6, at least 7, at least 8, at least 9, at least 10, or at least 15 of the other biomarkers listed in Tables 5 and 6.

In one embodiment, the methods involve determining the levels of a set of biomarkers, wherein the set of biomarkers includes at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes at least 2, at least 3, at least 4, or at least 5 of the other biomarkers listed in Tables 4 and 5.

In one embodiment, the methods involve determining the levels of a set of biomarkers, wherein the set of biomarkers includes at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|178779|gb|AAA51748.1 (Apolipoprotein A-IV) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|178779|gb|AAA51748.1 (Apolipoprotein A-IV) and at least 1, at least 2, at least 3, or at least 4 of the other biomarkers listed in Tables 4 and 5.

In one embodiment, the methods involve determining the levels of a set of biomarkers, wherein the set of biomarkers includes gi|3337390|gb|AAC27432.1 (Haptoglobin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|3337390|gb|AAC27432.1 (Haptoglobin) and at least 1, at least 2, at least 3, or at least 4 of the other biomarkers listed in Tables 4 and 5.

In one embodiment, the methods involve determining the levels of a set of biomarkers, wherein the set of biomarkers includes gi|182440|gb|AAB59530.1 (Fibrinogen gamma prime chain) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|182440|gb|AAB59530.1 (Fibrinogen gamma prime chain) and at least 1, at least 2, at least 3, or at least 4 of the other biomarkers listed in Tables 4 and 5.

In one embodiment, the methods involve determining the levels of a set of biomarkers, wherein the set of biomarkers includes gi|223002|prf∥0401173A (Fibrin beta) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|223002|prf∥0401173A (Fibrin beta) and at least 1, at least 2, at least 3, or at least 4 of the other biomarkers listed in Tables 4 and 5.

In one embodiment, the methods involve determining the levels of a set of biomarkers, wherein the set of biomarkers includes gi|4558178|pdb|1QAB|D (Retinol Binding Protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|4558178|pdb|1QAB|D (Retinol Binding Protein) and at least 1, at least 2, at least 3, or at least 4 of the other biomarkers listed in Tables 4 and 5.

In one embodiment, the methods involve determining the levels of a set of biomarkers, wherein the set of biomarkers includes P01011 (Alpha-1 antichymotrypsin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes P01011 (Alpha-1 antichymotrypsin) and at least 1, at least 2, at least 3, or at least 4 of the other biomarkers listed in Tables 4 and 5.

In one embodiment, the methods involve determining the levels of a set of biomarkers, wherein the set of biomarkers includes NP_000925.1 (Alpha-2 antiplasmin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes NP_000925.1 (Alpha-2 antiplasmin) and at least 1, at least 2, at least 3, or at least 4 of the other biomarkers listed in Tables 4 and 5.

In one embodiment, the methods involve determining the levels of a set of biomarkers, wherein the set of biomarkers includes gi|7023232|dbj|BAA91891.1 (Unnamed protein product) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|7023232|dbj|BAA91891.1 (Unnamed protein product) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|28373620|pdb|1MA9|A (Vitamin D binding protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|28373620|pdb|1MA9|A (Vitamin D binding protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|40786791|gb|AAR89906.1 (Complement component 3) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|40786791gb|AAR89906.1 (Complement component 3) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|5174525|ref|NP_005900.1 (Microtubule-associated protein 1B isoform 1) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|5174525|ref|NP_005900.1 (Microtubule-associated protein 1B isoform 1) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|8928566|sp|Q02952|AK12_ HUMAN (A-kinase anchor protein 12 (AKAP 250) (Myasthenia gravis autoantigen gravin)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|8928566|sp|Q02952|AK12_ HUMAN (A-kinase anchor protein 12 (AKAP 250) (Myasthenia gravis autoantigen gravin)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|22218654|pdb|1GPZ|B (Complement component 1, r subcomponent) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|22218654|pdb|1GPZ|B (Complement component 1, r subcomponent) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|15099973|gb|AAK84185.1 (Ig heavy chain variable region (anti-thrombospondin)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|15099973|gb|AAK84185.1 (Ig heavy chain variable region (anti-thrombospondin)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|41406064|ref|NP_005955.1 (Myosin, heavy polypeptide 10, non-muscle) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|41406064|ref|NP_005955.1 (Myosin, heavy polypeptide 10, non-muscle) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|17318569|ref|NP_006112.2 (Keratin 1) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|17318569|ref|NP_006112.2 (Keratin 1) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|386854|gb|AAA36153.1 (Keratin type II subunit protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|386854|gb|AAA36153.1 (Keratin type II subunit protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|1346640|sp|P35580|MYHA_HUMAN (Myosin heavy chain, nonmuscle type B) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|1346640|sp|P35580|MYHA_HUMAN (Myosin heavy chain, nonmuscle type B) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|22761659|dbj|BAC11646.1 (Unnamed protein product) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|22761659|dbj|BAC11646.1 (Unnamed protein product) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|544379|sp|P35574|GDE_RABIT (Glycogen debranching enzyme (Glycogen debrancher)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|544379|sp|P35574|GDE_RABIT (Glycogen debranching enzyme (Glycogen debrancher)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|4838009|gb|AAD30796.1 (Ig heavy chain variable region) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|4838009|gb|AAD30796.1 (Ig heavy chain variable region) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|1154664|emb|CAA62603.1 (Ataxia telangectasia mutated (A-T)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|1154664|emb|CAA62603.1 (Ataxia telangectasia mutated (A-T)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|34365215|emb|CAE45949.1 (TNN13-interacting kinase (FPGT-encoded)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|34365215|emb|CAE45949.1 (TNN13-interacting kinase (FPGT-encoded)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|339685|gb|AAA61181.1 (Transthyretin) and at least 1, at least 2, at least 3, at least 4, at least 5, at least 6, at least 7, at least 8, at least 9, at least 10, or at least 15 of the other biomarkers listed in Tables 4, 5 and 6

In one embodiment, the methods involve determining the levels of a set of biomarkers, wherein the set of biomarkers includes gi|339685|gb|AAA61181.1 (Transthyretin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|31615331|pdb|1HK3|A (Serum Albumin (mutant R218p)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|31615331|pdb|1HK3|A (Serum Albumin (mutant R218p)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|18044197|gb|AAH20197.1 (1-aminocyclopropane-1-carboxylate synthase) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|18044197|gb|AAH20197.1 (1-aminocyclopropane-1-carboxylate synthase) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|546095|gb|AAB30327.1 (Ig heavy chain variable region (anti-histone H1 WRI-170 antibody)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|546095|gb|AAB30327.1 (Ig heavy chain variable region (anti-histone H1 WRI-170 antibody)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|51476396|emb|CAH18188.1 (Alpha-2 macroglobulin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|51476396|emb|CAH18188.1 (Alpha-2 macroglobulin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes P06727 (Apolipoprotein A-IV) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes P06727 (Apolipoprotein A-IV) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|51467959|ref|XP_497224.1 (BMS1-like (similar to KIAA0187; ribosomal)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|51467959|ref|XP_497224.1 (BMS1-like (similar to KIAA0187; ribosomal)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|39573703|dbj|BAC19850.2 (complement component 1, r subcomponent) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|39573703|dbj|BAC19850.2 (complement component 1, r subcomponent) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|23273927|gb|AAH35014.1 (NudC domain containing 3 (KIAA1068 protein)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|23273927|gb|AAH35014.1 (NudC domain containing 3 (KIAA1068 protein)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|34526199|dbj|BAC85198.1 (Unnamed protein product) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|34526199|dbj|BAC85198.1 (Unnamed protein product) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes P25311 (Zinc alpha-2 glycoprotein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes P25311 (Zinc alpha-2 glycoprotein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|28466999|ref|NP_787057.1 (ARG99 protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|28466999|ref|NP_787057.1 (ARG99 protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|404108|dbj|BAA03864.1 (Glutathione peroxidase) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|404108|dbj|BAA03864.1 (Glutathione peroxidase) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|50255295|gb|EAL18030.1 (Hypothetical protein CNBK0510) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|50255295|gb|EAL18030.1 (Hypothetical protein CNBK0510) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|7428606|pir∥MHHUM (Ig mu chain C region, membrane-bound splice form) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|7428606|pir∥MHHUM (Ig mu chain C region, membrane-bound splice form) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|38649048|gb|AAH62986.1 (ZFR protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|38649048|gb|AAH62986.1 (ZFR protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|37790798|gb|AAR03501.1 (Angiotensinogen, member 8) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|37790798|gb|AAR03501.1 (Angiotensinogen, member 8) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|24308400|ref|NP_612366.1 (Chromosome 10 open reading frame 42) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|24308400|ref|NP_612366.1 (Chromosome 10 open reading frame 42) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|14625439|dbj|BAB61902.1 (KIAA1774 protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|14625439|dbj|BAB61902.1 (KIAA1774 protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|47124562|gb|AAH70299.1 (Haptoglobin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|47124562|gb|AAH70299.1 (Haptoglobin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|539627|pir∥A46546 (Leukocyte common antigen long splice form) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|539627|pir∥A46546 (Leukocyte common antigen long splice form) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|4557225|ref|NP_000005.1 (Alpha-2 macroglobulin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|4557225|ref|NP_000005.1 (Alpha-2 macroglobulin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|14600217|gb|AAK71298.1 (TCR beta chain Vbeta13S3) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|14600217|gb|AAK71298.1 (TCR beta chain Vbeta13S3) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|114006|sp|P06727|APA4_HUMAN (Apolipoprotein A-IV) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|114006|sp|P06727|APA4_HUMAN (Apolipoprotein A-IV) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|40788364|dbj|BAA34505.2 (KIAA0785 protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|40788364|dbj|BAA34505.2 (KIAA0785 protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|7023207|dbj|BAA91880.1 (BTB and kelch domain containing 4 protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|7023207|dbj|BAA91880.1 (BTB and kelch domain containing 4 protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|862457|dbj|BAA03941.1 (Enoyl-CoA hydratase/3-hydroxyacyl-CoA dehydrogenase alpha-subunit of trifunctional protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|862457|dbj|BAA03941.1 (Enoyl-CoA hydratase/3-hydroxyacyl-CoA dehydrogenase alpha-subunit of trifunctional protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|7022921|dbj|BAA91769.1 (LEPREL1 protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|7022921|dbj|BAA91769.1 (LEPREL1 protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|10437767|dbj|BAB15104.1 (Zinc finger protein 2) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|10437767|dbj|BAB15104.1 (Zinc finger protein 2) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|21071030|ref|NP_57602.2 (Alpha 1B glycoprotein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|21071030|ref|NP_570602.2 (Alpha 1B glycoprotein) and at least 1, at least 2, at least 3, at least 4, at least 5, at least 6, at least 7, at least 8, at least 9, at least 10, or at least 15 of the other biomarkers listed in Table 6

In one embodiment, the methods involve determining the levels of a set of biomarkers, wherein the set of biomarkers includes gi|28207863|emb|CAD62585.1 (Alpha-1 antitrypsin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|28207863|emb|CAD62585.1 (Alpha-1 antitrypsin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarker, wherein the set of biomarkers includes gi|51471047|ref|XP_370690.3 (AT rich interactive domain 2 (ARID, RFX-like)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|51471047|ref|XP_370690.3 (AT rich interactive domain 2 (ARID, RFX-like)) and at least 1, at least 2, at least 3, at least 4, at least 5, at least 6, at least 7, at least 8, at least 9, at least 10, or at least 15 of the other biomarkers, listed in Table 6.

In one embodiment, the methods involve determining the levels of a set of biomarkers, wherein the set of biomarkers includes gi|6470150|gb|AAF13605.1 (BiP protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|6470150|gb|AAF13605.1 (BiP protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|11138513|gb|AAG31421.1 (FcRn alpha chain) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|1138513|gb|AAG31421.1 (FcRn alpha chain) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|10120958|pdb|1EXU|A (MHC-related Fc Receptor) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|10120958|pdb|1EXU|A (MHC-related Fe Receptor) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|37747855|gb|AAH59367.1 (Transferrin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|37747855|gb|AAH59367.1 (Transferrin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|339486|gb|AAA61148.1 (Transferrin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|339486|gb|AAA61148.1 (Transferrin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|5817245|emb|CAB53743.1 (dJ20N2.2 (novel protein similar to tubulin, beta polypeptide 4, member Q (TUBB4Q)) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|5817245|emb|CAB53743.1 (dJ20N2.2 (novel protein similar to tubulin, beta polypeptide 4, member Q (TUBB4Q)) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|51476755|emb|CAH18343.1 (Hypothetical protein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|51476755|emb|CAH18343.1 (Hypothetical protein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|50363219|ref|NP_01002236.1 (Alpha-1 antitrypsin, member 1) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|50363219|ref|NP_01002236.1 (Alpha-1 antitrypsin, member 1) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|28566306|gb|AAO43053.1 (Heat shock regulated-1) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|28566306|gb|AAO43053.1 (Heat shock regulate-1) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|7428607|pir∥MHHU (Ig mu chain C region, secreted splice form) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|7428607|pir∥MHHU (Ig mu chain C region, secreted splice form) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|1703025|sp|P01009 (Alpha-1 antitrypsin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|1703025|sp|P01009 (Alpha-1 antitrypsin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|72059|pir∥NBHUA2 (Leucine-rich alpha-2-glycoprotein) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|72059|pir∥NBHUA2 (Leucine-rich alpha-2-glycoprotein) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|33469917|ref|NP_877423.1 (Minichromosome maintenance protein 4) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|33469917|ref|NP_877423.1 (Minichromosome maintenance protein 4) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|6650772|gb|AAF22007.1 (Serotransferrin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|6650772|gb|AAF22007.1 (Serotransferrin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|553788|gb|AAA61141.1 (Transferrin) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|553788|gb|AAA61141.1 (Transferrin) and at least 1, at least 2, at least 3, at least 4, at least 5, at least 6, at least 7, at least 8, at least 9, at least 10, or at least 15 of the other biomarkers listed in Table 6.

In one embodiment, the methods involve determining the levels of a set of biomarkers, wherein the set of biomarkers includes gi|47077177|dbj|BAD18510.1 (ZNF438 variant 3) and at least 1, at least 2, at least 3, at least 4, at least 5, at least 6, at least 7, at least 8, at least 9, at least 10, or at least 15 of the other biomarkers listed in Tables 4, 5 and 6.

In one embodiment, the methods involve determining the levels of a set of biomarkers, wherein the set of biomarkers includes gi|47077177|dbj|BAD18510.1 (ZNF438 variant 3) and at least 1, at least 2, at least 3, at least 4, at least 5, at least 6, at least 7, at least 8, at least 9, at least 10, or at least 15 of the other biomarkers listed in Table 6.

### VIII. EXAMPLES

### Example 1

### Characterization of Surrogate Biomarkers Associated with Age-related Macular Degeneration

### Introduction

Inflammation and/or immune-mediated processes, and particularly the alternative complement pathway, are strongly associated with the development of AMD (Hageman et al., Am J Ophthalmol. 132:411, 2002). Components of complement and immune-mediated pathways accumulate within drusen, the hallmark ocular deposits associated with AMD; many of these molecules are synthesized locally by the retinal pigment epithelium and choroid. Recently, a highly significant association between variations in the Factor H gene (CFH), which encodes a key regulator of the alternative complement pathway, and AMD have been documented. These data confirm and highlight the critical role of inflammation in AMD (Hageman et al., Proc Natl Acad Sci USA 102:7227, 2005).

We used serum proteome profiling to identify surrogate serum biomarkers for AMD by analyzing 20 pairs of serum/plasma samples obtained from two independent groups of AMD cases and age-matched controls.

### Materials and Methods

Individuals of European-American descent, over the age of 60, were enrolled under UI IRB approved protocols. Sera were collected under rigorous criteria and analyzed by 2-Dimensional Difference Gel Electrophoresis (DIGE), in combination with MALDI-ToF mass spectroscopy. 50 µg protein from pairs of samples (one control and one AMD) and a pooled internal standard were labeled using CyDye DIGE Fluor minimal dyes (Cy3, Cy5 and Cy2 respectively). The mixture, along with approximately 300 µg unlabelled pooled sample (for spot picking) was first separated on an immobiline IPG strip pH 3-10 via iso-electric focusing. Samples on the IPG strip were then reduced and alkylated followed by 2nd dimension gel separation on a 25x20 cm TRIS-Gly 12.5% gel using the Ettan DALT Six system. Separated gel spots were imaged using a TYPHOON 9400 laser scanner at 3 different wavelengths corresponding to the 3 dyes used to label samples (see Figure 1). Unlabeled protein spots were post-stained with Deep Purple fluorescent dye and imaged at a different wavelength. Ettan DeCyder software was used to obtain accurate quantification of differences between the samples, with an associated statistical significance.

### Results

A total of approximately 1,800 spots were detected in the gel depicted in Figure 2. Statistics were performed on gel spot volumes comparing serum proteins from AMD patients and control individuals. 90 protein spots showed significant t-test differences with p < 0.01. The average ratios between the fluorescent data ranged from 1.2-128 between AMD patients and control individuals.

Of the spots exhibiting significant differences between control individuals and AMD patients, 36 of the most significant proteins were excised from the pick gel using an Ettan Spot Picker (see Figures 3 and 4). Automated spot matching was employed to compare the distribution of spots between multiple gels. Spots were identified that were reliably and specifically present/absent or increased/reduced in the sera/plasma of patients with AMD compared to sera/plasma from patients without AMD. Protein identifications were made by matching the acquired peptide MS spectra to theoretical spectra generated from protein data bases (SwissProt). Thirty of 36 proteins were identified via MALDI-TOF peptide mass fingerprinting analysis with expectation < 0.05 (see Table 1) The proteins in Table 1 were classified according to their structure and/or function (*e.g.,* complement related protein, immune related protein, structural protein, enzyme, or protein of unknown or undetermined function).

**Table 1**

| IDENTIFICATION OF PROTEINS PRESENT AT SIGNIFICANTLY DIFFERENT LEVELS IN SERA OF AMD PATIENTS COMPARED TO CONTROLS | |
|---|---|
| Protein Spots Identified from Gel in Figure 2 (highest differential scores only)* | |
| 1. CAD62585.1 - | unnamed protein product⁵ |
| 2. CAH18188.1 - | hypothetical protein⁵ |
| 3. NP_000005.1 - | alpha-2-macroglobulin precursor³ |
| 4. NP_001002236.1- | serine (or cysteine) proteinase inhibitor, clade A (alpha-1 |
| | antiproteinase, antitrypsin), member 1; protease inhibitor 1 (anti- |
| | elastase), alpha-1-antitrypsin³ |
| 5. AAR89906.1 - | complement component 3¹ |
| 6. CAH18188.1 - | hypothetical protein⁵ |
| 7. CAH18343.1 - | hypothetical protein⁵ |
| 8. CAH18343.1 - | hypothetical protein⁵ |
| 9. BAC19850.2 - | r subcomponent of complement component 1¹ |
| 10. CAB53743.1 - | dJ20N2.2 (novel protein similar to tubulin, beta polypeptide 4, member Q (TUBB4Q))³ |
| 11. MHHUM - | Ig mu chain C region, membrane-bound splice form² |
| 12. MHHU - | Ig mu chain C region, secreted splice form² |
| 13.AAA61141.1- | transferrin³ |
| 14. BAA03941.1 - | enoyl-CoA hydratase/3-hydroxyacyl-CoA dehydrogenase alpha-subunit of trifunctional protein⁴ |
| 15. BAC11646.1 - | unnamed protein product⁵ |
| 16. NP_000925.1 - | alpha-2-plasmin inhibitor; alpha-2-antiplasmin³ |
| 17. AAH59367.1 - | Transferrin³ |
| 18. NP_570602.2 - | alpha 1B-glycoprotein³ |
| 19. AAR03501.1 - | angiotensinogen (serine (or cysteine) proteinase inhibitor, clade A (alpha-1 antiproteinase, antitrypsin), member 8)³ |
| 20. BAC19850.2 - | r subcomponent of complement component 1¹ |
| 21. 1MA9\|A - | Chain A, Crystal Structure Of The Complex Of Human Vitamin D Binding Protein And Rabbit Muscle Actin² |
| 22. AAK71298.1- | TCR beta chain Vbeta13S3-VAGARNTEAFF-Jbetal.1² |
| 23. NBHUA2 - | leucine-rich alpha-2-glycoprotein³ |
| 24. APA4_HUMAN- | Apolipoprotein A-IV precursor³ |
| 25. AAH70299.1 - | HP protein² |
| 26. AAD30796.1 - | immunoglobulin heavy chain variable region² |
| 27. AAK84185.1 - | anti-thrombospondin immunoglobulin heavy chain variable region² |
| 28. 1GPZ\|B - | Chain B, The Crystal Structure Of The Zymogen Catalytic Domain Of Complement Protease C1¹ |
| 29. NP_006112.2 - | keratin 1; (Keratin-1; cytokeratin 1; hair alpha protein³ |
| 30. AAK84185.1 - | anti-thrombospondin immunoglobulin heavy chain variable region² |
| 31. BAA34505.2 - | KIAA0785 protein⁵ |
| 32. NP_005955.1 - | myosin, heavy polypeptide 10, non-muscle; myosin heavy chain, nonmuscle type B; cellular myosin heavy chain, type B³ |
| 33. NF_787057.1 - | ARG99 protein³ |
| 34. AAH20197.1 - | 1-amrnocyclopropane-1-carboxylate synthase⁴ |
| 35. AAK84185.1 - | anti-thrombospondin immunoglobulin heavy chain variable region² |
| 36. AAA61181.1 - | transthyretin³ |

| | |
|---|---|
| *Proteins in this table were classified according to their structure and/or function: ¹complement related protein; ²immune related protein; ³structural protein; ⁴enzyme; ⁵protein of unknown or undetermined function. | |

### Example 2

### IDENTIFICATION OF PROTEINS PRESENT AT DIFFERENT LEVELS IN SERA FROM AMD PATIENTS COMPARED TO CONTROL INDIVIDUALS

Sera from AMD patients and age-matched control individuals were analyzed by 2-dimensional difference gel electrophoresis (DIGE) followed by protein identification by MALDI-ToF mass spectroscopy as described in Example 1. The image of proteins separated by DIGE depicting the spots/proteins picked using an Ettan Spot Picker are shown in Figures 5 and 6. Each spot/protein that was picked for quantification was given a master spot number as indicated in the figures and tables below. The identity of proteins corresponding to each master spot number in Figures 5 and 6 is shown below in Tables 2 and 3, respectively.

**Table 2**

| MALDI REPORT OF PROTEIN IDENTIFICATION FOR SPOTS IN THE DIGE SHOWN IN FIGURE 5 | | | | | | | | |
|---|---|---|---|---|---|---|---|---|
| Pos. | Master Spot no. | Method | Rank | Expect. | Protein information | Cov. | pI | Mass [kDa] |
| 1 | 212 | Sample 1 | 1 | 0.000 | gi\|1942284\|pdb\|1KCW - X-Ray Crystal Structure Of Human Ceruloplasmin At 3.0 Angstroms | 32.8 | 5.4 | 120.87 |
| 2 | 222 | Sample 2 | 1 | 0.000 | gi\|1942284\|pdb\|1KCW - X-Ray Crystal Structure Of Human Ceruloplasmin At 3.0 Angstroms | 25.9 | 5.4 | 120.87 |
| | | | 2 | 0.198 | gi\|38568178\|ref\|MP_056099.1 -KIAA0467 protein [Homo saplens] | 6.3 | 6.3 | 219.86 |
| | | | 3 | 0.339 | gi\|7512684\|pir∥T34543 - hypothetical protein DKFZp43401221.1 - human (fragment) gi\|6102944\|emb\|CAB59276.1\| hypothetical protein [Homo sapiens] | 18.5 | 9.7 | 42.08 |
| 3 | 704 | Sample 3 | 1 | 0.000 | gi\|69990\|pir∥OMHU1B - alpha-1-B-glycoprotein - human | 33.1 | 5.6 | 52.49 |
| | | | 2 | 0.025 | gi\|14488709\|pdb\|1E33\|P - Chain P, Crystal Structure Of An Arylsulfatase A Mutant P4261 | 17.6 | 5.5 | 52.61 |
| | | | 3 | 0.043 | gi\|4505313\|ref\|NP_003794.1-myomesin 1; myomesin (M-protein) 1 (165kD); myomesin (M-protein) 1 (190kD); myomesin 1 (skelemin) (185kD); skelemin; EH-myomesin [Homo sapiens] gi\|1709202\|sp\|P52179\|MYM1_HUMAN Myomesin 1 (190 kDa titin-associated protein) (190 kDa connectin-associated protein) gi\|631050\|pir∥S42167 190K protein - human gi\|407099\|emb\|CAA48833.1\| 190kD protein [Homo sapiens] | 7.2 | 6.1 | 163.80 |
| 4 | 730 | Sample 4 | 1 | 0.000 | gi\|69990\|pir∥OMHU1B - alpha-1-B-glycoprotein - human | 34.8 | 5.6 | 52.49 |
| | | | 2 | 0.426 | gi\|28872776\|ref\|NP_788274.1-DNA methyltransferase 2 isoform f; DNA methyltransferase-2; DNA methyltransferase homolog HsaIIP; DNA MTase homolog HsaIIP [Homo sapiens] | 24.8 | 9.6 | 13.15 |
| 5 | 778 | Sample 5 | 1 | 0.000 | gi\|4557871\|ref\|NP_001054.1 -transferrin [Homo sapiens] | 35.7 | 6.9 | 79.31 |
| | | | 2 | 0.321 | gi\|21748558\|dbj\|BAC03416.1 -FLJ00353 protein [Homo saplens] | 7.5 | 6.2 | 200.78 |
| 6 | 785 | Sample 6 | 1 | 0.000 | gi\|11386143\|ref\|NP_000925.1 - alpha-2-plasmin Inhibitor; alpha-2-antiplasmin [Homo sapiens] | 25.5 | 5.8 | 54.92 |
| | | | 2 | 0.388 | gi\|12232415\|ref\|NP_073588.1 -chromosome 18 open reading frame 11 [Homo sapiens] gi\|10437739\|dbj\|BAB15094.1\| unnamed protein product [Homo sapiens] | 13.5 | 6.3 | 98.51 |
| 7 | 810 | Sample 7 | 1 | 0.000 | gi\|31615330\|pdb\|lHK2\|A - Chain A, Human Serum Albumin Mutant R218h Complexed With Thyroxine (3,3',5,5'-Tetralodo-L-Thyronine) | 35.0 | 5.7 | 68.43 |
| | | | 2 | 0.003 | gi\|15679996\|gb\|AAH14308.1-Unknown (protein for IMAGE:3934797) [Homo sapiens] | 41.3 | 8.6 | 23.32 |
| | | | 3 | 0.239 | gi\|34740327\|ref\|NP_919226.1 - similar to C630007C17Rik protein [Homo sapiens] gi\|33087209\|gb\|AAP92799.1\| hypothetical protein [Homo sapiens] | 8.3 | 8.7 | 138.70 |
| 8 | 825 | Sample 8 | 1 | 0.000 | gi\|31615331\|pdb\|1HK3\|A - Chain A, Human Serum Albumin Mutant R218p Complexed With Thyroxine (3,3',5,5'-Tetralodo-L-Thyronine) | 40.7 | 5.6 | 68.39 |
| | | | 2 | 0.149 | gi\|15679996\|gb\|AAH14308.1 - Unknown (protein for IMAGE:3934797) [Homo sapiens] | 35.3 | 8.6 | 23.32 |
| | | | 3 | 0.256 | gi\|34740327\|ref\|NP_919226.1-simllar to C630007C17Rlk protein [Homo sapiens] gi\|33087209\|gb\|AAP92799.1\| hypothetical protein [Homo sapiens] | 9.9 | 8.7 | 138.70 |
| 9 | 857 | Sample 9 | 1 | 0.000 | gi\|31615331\|pdb\|1HK3\|A - Chain A, Human Serum Albumin Mutant R218p Complexed With Thyroxine (3,3',5,5'-Tetralodo-L-Thyronine) | 40.3 | 5.6 | 68.39 |
| | | | 2 | 0.001 | gi\|15679996\|gb\|AAH14308.1 - Unknown (protein for IMAGE:3934797) [Homo sapiens] | 51.7 | 8.6 | 23.32 |
| 10 | 863 | Sample 10 | 1 | 0.034 | gi\|443345\|pdb\|2ACH\|A - Chain A, Alpha1 Antichymotrypsin | 21.7 | 5.1 | 40.70 |
| | | | 2 | 0.049 | gi\|112874\|sp\|P01011\|AACT_HUMAN -Alpha-1-antichymotrypsin precursor (ACT) gi\|13097705\|gb\|AAH03559.1\| SERPINA3 protein [Homo sapiens] gi\|14714766\|gb\|AAH10530.1\| SERPINA3 protein [Homo sapiens] | 16.8 | 5.3 | 47.80 |
| | | | 3 | 0.465 | gi\|33469951\|ref\|NP_878256.1 - Rab geranylgeranyltransferase, alpha subunit isoform a [Homo sapiens] gi\|6093707\|sp\|Q92696\|PGTA_HUMAN Geranylgeranyl transferase type II alpha subunit (Rab geranylgeranyltransferase alpha subunit) (Rab geranyl-geranyltransferase alpha subunit) (Rab GG transferase alpha) (Rab GGTase alpha) gi\|7513291\|pir∥JC5538 Rab geranylgeranyl transferase (EC 2.5.1.-) alpha chain - human gi\|2950170\|emb\|CAA69382.1\| rab geranylgeranyl transferase [Homo sapiens] gi\|13111853\|gb\|AAH03093.1\| Rab geranylgeranyltransferase, alpha subunit, isoform a [Homo sapiens] | 14.6 | 5.5 | 66.14 |
| 11 | 893 | Sample 11 | 1 | 0.120 | gi\|21450669\|ref\|NP_659417.1 -chromosome 6 open reading frame 118 [Homo sapiens] | 15.4 | 8.8 | 54.40 |
| 12 | 942 | Sample 12 | 1 | 0.000 | gi\|999514\|pdb\|1ATH\|B - Chain B, Antithrombin Ill | 34.0 | 6.0 | 49.27 |
| | | | 2 | 0.000 | gi\|34526199\|dbj\|BAC85198.1 -unnamed protein product [Homo sapiens] | 27.9 | 7.9 | 54.34 |
| | | | 3 | 0.053 | gi\|229537\|prf∥752400A - Ig A H | 22.7 | 10.0 | 52.08 |
| 13 | 1016 | Sample 13 | 1 | 0.000 | gi\|1703025\|sp\|P01009\|A1AT_HUMAN -Alpha-1-antitrypsin precursor (Alpha-1 protease Inhibitor) (Alpha-1-antiproteinase) (PRO0684/PRO2209) | 37.8 | 5.4 | 46.89 |
| 14 | 1039 | Sample 14 | 1 | 0.000 | gi\|28207863\|emb\|CAD62585.1 -unnamed protein product [Homo sapiens] | 24.9 | 5.1 | 41.60 |
| | | | 2 | 0.031 | gi\|35493719\|ref\|NP_908931.1 - Cohen syndrome 1 protein isoform 2 [Homo sapiens] | 9.4 | 5.5 | 161.94 |
| | | | 3 | 0.083 | gi\|177827\|gb\|AAA51546.1 - alpha-1-antitrypsin | 18.0 | 5.4 | 46.80 |
| 15 | 1100 | Sample 15 | - | - | - | - | - | - |
| 16 | 1103 | Sample 16 | 1 | 0.001 | gi\|223002\|prfi\|0401173A - fibrin beta | 32.0 | 8.3 | 51.37 |
| | | | 2 | 0.172 | gi\|31565122\|gb\|AAH53521.1 - SPTAN1 protein [Homo sapiens] | 6.9 | 5.2 | 283.06 |
| | | | 3 | 0.194 | gi\|29421212\|dbj\|BAC02706.2 -KIAA1997 protein [Homo sapiens] | 9.3 | 6.3 | 192.86 |
| 17 | 1120 | Sample 17 | - | - | - | - | - | - |
| 18 | 1135 | Sample 18 | 1 | 0.039 | gi\|29421212\|dbj\|BAC02706.2 -KIAA1997 protein [Homo sapiens] | 6.5 | 6.3 | 192.86 |
| 19 | 1137 | Sample 19 | 1 | 0.000 | gi\|28373620\|pdb\|1MA91A - Chain A, Crystal Structure Of The Complex Of Human Vitamin D Binding Protein And Rabbit Muscle Actin | 53.3 | 5.2 | 52.81 |
| | | | 2 | 0.297 | gi\|35493719\|ref\|NP_908931.1 - Cohen syndrome 1 protein Isoform 2 [Homo sapiens] | 8.0 | 5.5 | 161.94 |
| 20 | 1147 | Sample 20 | 1 | 0.000 | gi\|18655424\|pdb\|1378\|A-Chain A, Crystallographic Analysis Of The Human Vitamin D Binding Protein | 51.5 | 5.2 | 52.80 |
| | | | 2 | 0.187 | gi\|14165405\|ref\|NP_113683.1 -protocadherin alpha 2 isoform 2 precursor; KIAA0345-like 12 [Homo sapiens] gi\|3540168\|gb\|AAC34324.1\| KIAA0345-like 12 [Homo sapiens] gi\|5457009\|gb\|AAD43741.1\| protocadherin alpha 2 short form protein [Homo sapiens] | 10.7 | 4.9 | 89.36 |
| 21 | 11481 | Sample 21 | 1 | 0.000 | gi\|18655424\|pdb\|1378\|A-Chain A, Crystahographte Analysis Of The Human Vitamin D Binding Protein | 53.3 | 5.2 | 52.80 |
| 22 | 1203 | Sample 22 | 1 | 0.006 | gi\|4503715\|ref\|MP_000500.1-fibrinogen, gamma chain isoform gamma-A precursor [Homo sapiens] | 24.9 | 5.6 | 50.09 |
| | | | 2 | 0.257 | gi\|22766790\|gb\|AAH32944.1 - Similar to ubiqultin-ligase [Homo sapiens] | 14.3 | 7.8 | 91.23 |
| 23 | 1245 | Sample 23 | 1 | 0.000 | gl\|71827\|pir∥FGHUG - fibrinogen gamma-A chain precursor [valldated] -human | 51.5 | 5.7 | 50.11 |
| | | | 2 | 0.332 | gi\|3667040\|ref\|NP_789781.2-NACHT, leucine rich repeat and PYD containing 8; NACHT, LRR and PYD containing protein 8 [Homo sapiens] gi\|30348942\|tpg\|DAA01242.1\| TPA: NOD16 [Homo sapiens] | 10.6 | 9.1 | 121.92 |
| | | | 3 | 0.381 | gi\|20530939\|gb\|AAM27295.1 -phosphoglycerate mutase processed protein [Homo sapiens] | 35.0 | 6.0 | 28.90 |
| 24 | 1259 | Sample 24 | 1 | 0.000 | gi\|11761633\|ref\|NP_68656.1-fibrinogen, gamma chain isoform gamma-B precursor [Homo sapiens] | 49.7 | 5.3 | 52.11 |
| | | | | | | | | |
| 1 | 1292 | Sample 1 | 1 | 0.001 | gi\|337390\|gb\|AAC27432.1 -haptoglobin [Homo sapiens] | 26.7 | 6.1 | 38.73 |
| | | | 2 | 0.195 | gi\|38348290\|ref\|NP_940890.1 -FLJ46072 protein [Homo sapiens] gl\|34535099\|dbj\|BAC87207.1\| unnamed protein product [Homo sapiens] | 10.5 | 9.1 | 94.58 |
| 2 | 1360 | Sample 2 | 1 | 0.000 | gi\|178779\|gb\|AAA1748.1 -apolipoproteln A-IV precursor | 63.6 | 5.2 | 43.37 |
| | | | 2 | 0.333 | gi\|773575\|emb\|CAA57072.1 - archain [Homo sapiens] | 16.3 | 5.5 | 53.39 |
| | | | 3 | 0.450 | gi\|4520225\|dbj\|BM75636.1 - Rho kinase [Homo sapiens] | 7.3 | 5.8 | 162,01 |
| 3 | 1498 | Sample 3 | 1 | 0.154 | gi\|4028545\|gb\|AAC96300.1 - neural UM domain only 7 [Homo sapiens] | 80.4 | 12.3 | 5.20 |
| 4 | 1506 | Sample 4 | 1 | 0.018 | gl\|4502067\|ref\|NP_001624.1 - alpha-1-microglobulin/bikunin precursor; Alpha-1-microglobulin/bikunin precursor (inter-alpha-trypsin inhibitor, light chain; protein HC); Alpha-1-mlcroglobulin/bikunin precursor; Inter-alpha-trypsin [Homo sapiens] | 24.7 | 6.0 | 39.89 |
| 5 | 1513 | Sample 5 | 1 | 0.364 | gi\|2654365\|emb\|CAA77836.1 -selenoprotein P [Homo sapiens] | 27.3 | 8.4 | 42.63 |
| 6 | 1569 | Sample 6 | 1 | 0.049 | gi\|40786420\|ref\|NP_955383.1 - Clorf32 putative protein [Homo sapiens] | 13.5 | 8.9 | 72.15 |
| | | | 2 | 0.176 | gi\|41148476\|ref\|XP_372063.1 - similar to epiplakin [Homo sapiens] | 4.0 | 5.7 | 274.91 |
| | | | 3 | 0.248 | gi\|34530791\|dbj\|BAC85978.1-unnamed protein product [Homo sapiens] | 15.2 | 9.0 | 36.94 |
| 7 | 1723 | Sample 7 | 1 | 0.001 | gi\|78775\|gb\|AAA51747.1-proapolipoprotein | 43.8 | 5.4 | 28.94 |
| 8 | 1729 | Sample 8 | 1 | 0.001 | gi\|178775\|gb\|AAA51747.1 - proapolipoprotein | 44.6 | 5.4 | 28.94 |
| | | | 2 | 0.064 | gi\|i205990\|gb\|AA802621.1 - nebulin | 7.1 | 9.2 | 286.75 |
| | | | 3 | 0.232 | gi\|229479\|prf\|\|740525A - lipoprotein Gln I gi\|229513\|prf\|\|750843A protein,lipid binding AI | 28.2 | 5.3 | 28.33 |
| 9 | 1732 | Sample 9 | 1 | 0,000 | gi\|178775\|gb\|AAA51747.1 -proapolipoprotein | 49.0 | 5.4 | 28.94 |
| | | | 2 | 0.390 | gi\|20521001\|db\|BAA20786.3 -KIAA0328 protein [Homo sapiens] | 5.3 | 8.5 | 232.19 |
| 10 | 1740 | Sample 10 | 1 | 0.080 | gi\|14165456\|ref\|NP\|-114399.1 - microtubule-associated protein 1B isoform 2 [Homo sapiens] | 6.0 | 4.7 | 257.83 |
| | | | 2 | 0.098 | gl14503051\|ref\|NP_001304.1 - collapsin response mediator protein 1; collapsin response mediator protein 1 (dihydropyrimidinase-ilke 1) [Homo sapiens] gi\|3122031\|sp\|Q14194\|DPY1_HUMAN Dthydropyrimidinase related protein-1 (DRP-1) (Coilapsin response mediator protein 1) (CRMP-1) g\|17512386\|pir\|\|Jc5316 dlhydropyrimidinase related protein 1 -human gi\|1330238\|dbj\|BAA11190.1\| dihydropyrimidinase related protein-1 [Homo sapiens] gi\|12652983\|gb\|AA1400252.1\| Collapsin response mediator protein 1 [Homo sapiens] gi\|14043246\|gb\|AAH07613.1\| Collapsin response mediator protein 1 [Homo sapiens] gi\|30582451\|gb\|AAP35452.1\| coliapsin response mediator protein 1 [Homo sapiens] | 12.8 | 6.6 | 62.51 |
| | | | 3 | 0.209 | g\|14502511\|ref\|NP_001728.1 -complement component 9 [Homo sapiens] gi\|1352108\|sp\|P02748\|CO9_HUMAN Complement component C9 precursor gi\|25757818\|pir\|\|C9HU complement C9 precursor [validated] - human gi\|29581\|emb\|CAA26117.1\| unnamed protein product [Homo sapiens] gi\|18088821\|gb\|AAH20721.11 Complement component 9 [Homo sapiens] | 15.2 | 5.4 | 64.64 |
| 11 | 1742 | Sample 11 | 1 | 0.000 | gi\|230284\|pdb\|RBP - Retinol Binding Protein | 63.2 | 5.3 | 21.28 |
| | | | 2 | 0.075 | gi\|7657184\|ref\|NP_055160.1 - zinc finger protein 318; endocrine regulator [Homo sapiens] gi\|S712209\|gb\|AAD47387.1\| unknown [Homo sapiens] | 5.5 | 6.0 | 233.50 |
| 12 | 1771 | Sample 12 | 1 | 0.002 | gi\|4558176\|pdb\|1QAB\|B - Chain B, The Structure Of Human Retinol Binding Protein With Its Carrier Protein Transthyretin Reveals Interaction With The Carboxy Terminus Of Rbp | 61.0 | 5.5 | 12.98 |
| 13 | 1790 | Sample 13 | 1 | 0.210 | gi\|4559298\|gb\|AAD22973.1 - silencing mediator of retinoic acid and thyroid hormone receptor extended isoform [Homo sapiens] | 5.3 | 7.5 | 274.07 |

| | | | | | | | | |
|---|---|---|---|---|---|---|---|---|
| *Cov. (coverage) indicates the percentage of peptides that were identified by MALDI-ToF mass spectroscopy for each of the indicated proteins. | | | | | | | | |

**Table 3.**

| MALDI REPORT OF PROTEIN IDENTIFICATION FOR SPOTS IN THE DIGE SHOWN IN FIGURE 6 | | | | | | | | |
|---|---|---|---|---|---|---|---|---|
| Pos. | Master spot no. | Method | Rank | Expect. | Protein information | Cov. | pI | Mass [kDa] |
| 1 | 65 | Sample 1 | 1 | 0.006 | gi\|28207863\|emb\|CAD62585.1-unnamed protein product [Homo sapiens] | 19.7 | 5.1 | 41.60 |
| | | | 2 | 0.059 | gi\|10120958\|pdb\|1EXU\|A - Chain A, Crystal Structure Of The Human Mhc-Related Fc Receptor | 25.5 | 6.1 | 30.01 |
| | | | 3 | 0.113 | gi\|1138513\|gb\|AAG31421.1 - FcRn alpha chain [Homo sapiens] | 18.0 | 5.9 | 39.72 |
| 2 | 81 | Sample 2 | 1 | 0.000 | g\|151476396\|emb\|CAH18188.1 -hypothetical protein [Homo sapiens] | 8.0 | 6.0 | 164.72 |
| 3 | 106 | Sample 3 | 1 | 0.000 | gi\|4557225\|ref\|NP_00005.1 - alpha-2-macroglobulin precursor [Homo sapiens] | 13.7 | 6.0 | 164.69 |
| 4 | 111 | Sample 4 | 1 | 0.000 | gi\|50363219\|ref\|NP_001002236.1 -serine (or cysteine) proteinase inhibitor, clade A (alpha-1 antiproteinase, antitrypsin), member 1; protease inhibitor 1 (anti-elastase), alpha-1-antitrypsin [Homo sapiens] | 34.9 | 5.4 | 46.89 |
| | | | 2 | 0.375 | gi\|28566306\|gb\|AA043053.1 - heat shock regulated-1 [Homo sapiens] | 5.2 | 6.1 | 224.89 |
| 5 | 209 | Sample 5 | 1 | 0.044 | - gi\|40786791\|gb\|AAR89906.1 -complement component 3 [Homo sapiens] | 5.3 | 6.0 | 188.67 |
| | | | 2 | 0.061 | g!\|5174525\|ref\|NP_005900.1 -microtubule-assoclated protein 1B isoform 1 [Homo sapiens] gi\|1170875\|sp\|P46821\|MAPB_HUMAN Microtubule-associated protein 1B (MAP 1B) [Contains: MAP1 ilght chain LC1] gi\|473431\|gb\|AAA18904.1\| microtubule-associated protein 1B | 4.7 | 4.7 | 271.80 |
| | | | 3 | 0.118 | gi\|8928566\|sp\|Q02952\|AK12_HUMAN -A-kinase anchor protein 12 (A-kinase anchor protein 250 kDa) (AKAP 250) (Myasthenia gravis autoantigen gravin) gi\|2218077\|gb\|AAC51366.1\| gravin [Homo sapiens] | 5.3 | 4.4 | 191.99 |
| 6 | 235 | Sample 6 | - | - | | - | - | - |
| 7 | 256 | Sample 7 | 1 | 0.004 | gi\|51476396\|emb\|CAH18188.1 -hypothetical protein [Homo sapiens] | 7.1 | 6.0 | 164.72 |
| | | | 2 | 0.226 | gi\|1154664\|emb\|CAA62603.1 - A-T [Homo sapiens] | 6.2 | 6.4 | 159.31 |
| 8 | 322 | Sample 8 | 1 | 0.012 | gi\|51476755\|emb\|CAH18343.1 -hypothetical protein [Homo sapiens] | 12.9 | 5.9 | 81.70 |
| | | | 2 | 0.053 | gi\|14625439\|dbj\|BAB61902.1 - KIAA1774 protein [Homo sapiens] | 6.8 | 4.6 | 126.87 |
| | | | 3 | 0.200 | gi\|7022921\|dbj\|AA91769.1 - unnamed protein product [Homo sapiens] gi\|13477143\|gb\|AAH05029.1i\| LEPREL1 protein [Homo sapiens] | 12.9 | 5.0 | 61.05 |
| 9 | 332 | Sample 9 | 1 | 0.000 | gi\|51476755\|emb\|CAH18343.1 -hypothetical protein [Homo sapiens] | 20.4 | 5.9 | 81.70 |
| | | | 2 | 0.181 | gi\|38649048\|gb\|AAH62986.1 - ZFR protein [Homo sapiens] | 13.5 | 9.3 | 69.18 |
| 10 | 350 | Sample 10 | 1 | 0.007 | gi\|39573703\|db\|BAC19850.2 - r subcomponent of complement component 1 [Homo sapiens] | 11.8 | 5.9 | 81.72 |
| | | | 2 | 0.084 | gi\|14625439\|db\|BAB61902.1 - KIAA1774 protein [Homo sapiens] | 4.7 | 4.6 | 126.87 |
| 11 | 447 | Sample 11 | 1 | 0.278 | gi\|58\|7245\|emb\|CAB53743.1 - dJ20N2.2 (novel protein similar to tubulin, beta polypeptide 4, member Q (TUBB4Q)) [Homo sapiens] | 12.2 | 4.4 | 18.90 |
| | | | 2 | 0.410 | gi\|546095\|gb\|AAB30327.1-anti-histone H1 WM-170 antibody heavy chain variable region [human, systemic lupus erythematosus (Wri) patient, Peptide Partial, 98 aa] | 55.1 | 9.5 | 10.83 |
| 12 | 468 | Sample 12 | 1 | 0.012 | gi\|7428606\|pir\|\|MHHUM - Ig mu chain C region, membrane-bound splice form -human | 15.0 | 5.8 | 52.43 |
| 13 | 473 | Sample 13 | 1 | 0.000 | gi\|7428607\|pir\|\|MHHU - Ig mu chain C region, secreted splice form - human | 19.7 | 6.4 | 50.01 |
| 14 | 496 | Sample 14 | 1 | 0.000 | gi\|6650772\|gb\|AAF22007.1 - PRO1400 [Homo sapiens] | 23.1 | 7.0 | 65.33 |
| | | | 2 | 0.002 | gi\|553788\|gb\|AAA61141.1 - transferin | 18.6 | 6.0 | 55.23 |
| | | | 3 | 0.178 | gi\|33469917\|ref\|NP_877423.1-minichromosome maintenance protein 4; homolog of S. pombe cell devision cycle 21; DNA replication licensing factor MCM4; minichromosome maintenance deficient (S. cerevislae) 4 [Homo sapiens] gi\|3469919\|ref\|NP_005905.21 minichromosome maintenance protein 4; homolog of S. pombe cell devision cycle 21; DNA replication licensing factor MCM4; minichromosome maintenance deficient (S. cerevislae) 4 [Homo sapiens] | 6.7 | 6.3 | 97.11 |
| 15 | 500 | Sample 15 | 1 | 0.276 | gi\|862457\|db\|BAA03941.1 - enoyl-CoA hydratase/3-hydroxyacyl-CoA dehydrogenase alpha-subunit of trifunctional protein [Homo sapiens] | 13.8 | 9.4 | 83.68 |
| | | | 2 | 0.298 | gi\|10437767\|dbj\|BAB15104.1 - unnamed protein product [Homo sapiens] | 17.0 | 9.9 | 46.47 |
| | | | 3 | 0.431 | gi\|7023207\|dbj\|BAA91880.1 - unnamed protein product [Homo sapiens] gi\|32189385\|ref\|NP_057590.21 keich repeat and BTB (POZ) domain containing 4; BTB and keich domain containing 4 [Homo sapiens] gi\|47117914\|sp\|Q9NVX7\|KBT4_HUMAN Kelch repeat and BTB domain containing protein 4 (BTB and kelch domain containing protein 4) | 17.6 | 5.4 | 59.05 |
| 16 | 555 | Sample 16 | 1 | 0.031 | gi\|22761659\|dbj\|BAC11646.1 - unnamed protein product [Homo sapiens] | 30.2 | 5.6 | 24.91 |
| 17 | 572 | Sample 17 | 1 | 0.001 | gi\|11386143\|ref\|NP_000925.1 - alpha-2 plasmin inhibitor; alpha-2-antiplasmln [Homo sapiens] | 18.1 | 5.8 | 54.92 |
| | | | 2 | 0.027 | gi\|21071030\|ref\|NP_570602.2 - alpha IB-glycoprotein [Homo sapiens] gi\|51555785\|db\|BAD38648.1\| alpha-1-B glycoprotein precursor [Homo sapiens] | 20.6 | 5.6 | 54.81 |
| | | | 3 | 0.038 | gi\|6470150\|gb\|AAF13605.1 - BiP protein [Homo sapiens] | 18.2 | 5.2 | 71.03 |
| 18 | 581 | Sample 18 | 1 | 0.000 | gi\|37747855\|gb\|AAH59367.1 -Transferrin [Homo sapiens] | 30.9 | 7.1 | 79.34 |
| | | | 2 | 0.002 | gi\|339486\|gb\|AAA61148.1 - transferrin | 54.1 | 9.1 | 16.00 |
| | | | 3 | 0.147 | gi\|51471047\|ref\|XP_370690.3 -PREDICTED: AT rich interactive domain 2 (ARID, RFX-like) [Homo sapiens] | 6.9 | 9.3 | 215.12 |
| 19 | 601 | Sample 19 | 1 | 0.005 | gi\|21071030\|ref\|NP_570602.2 - alpha 1B-glycoprotein [Homo sapiens] | 24.4 | 5.6 | 54.81 |
| | | | 2 | 0.022 | gi\|6470150\|gb\|AAF13605.1 - BIP protein [Homo sapiens] | 21.8 | 5.2 | 71.03 |
| | | | 3 | 0.123 | gi\|28207863\|emb\|CAD62585.1 -unnamed protein product [Homo sapiens] | 20.8 | 5.1 | 41.60 |
| 20 | 872 | Sample 20 | 1 | 0.000 | gi\|37790798\|gb\|AAR03501.1-angiotensinogen (serine (or cysteine) proteinase inhibitor, clade A (alpha-1 antiproteinase, antitrypsin), member 8) [Homo sapiens] | 23.1 | 5.9 | 53.39 |
| | | | 2 | 0.103 | gi\|24308400\|ref\|NP-612366.1 -chromosome 10 open reading frame 42 [Homo sapiens] gi\|21707703\|gb\|AAH34235.1\| Chromosome 10 open reading frame 42 [Homo sapiens] | 21.9 | 9.1 | 40.13 |
| 21 | 876 | Sample 21 | 1 | 0.001 | gi\|39573703\|dbj\|BAC19850.2 -subcomponent of complement component 1 [Homo sapiens] | 16.2 | 5.9 | 81.72 |
| | | | 2 | 0.020 | gi\|23273927\|gb\|AAH35014.1 - KIAA1068 protein [Homo sapiens] | 24.7 | 5.1 | 40.88 |
| | | | 3 | 0.471 | gi\|51467959\|ref\|XP_497224.1 -PREDICTED: similar to KIAA0187 [Homo sapiens] | 16.2 | 9.6 | 59.06 |
| 22 | 946 | Sample 22 | 1 | 0.000 | gi\|28373620\|pdb\|1MA9\|A-Chain A. Crystal Structure Of The Complex Of Human. Vitamin D Binding Protein And Rabbit Muscle Actin | 25.1 | 5.2 | 52.81 |
| | | | 2 | 0.377 | gi\|7023232\|dbj\|BAA91891.1 - unnamed protein product [Homo sapiens] | 8.5 | 6.6 | 84.79 |
| 23 | 952 | Sample 23 | - | - | - | - | - | - |
| 24 | 958 | Sample 24 | 1 | 0.332 | gi\|14600217\|gbl\|AK71298.1 - TCR beta chain Vbeta13S3-VAGARMTEAFF-Jbetal.11 [Homo sapiens] | 45.3 | 5.7 | 16.65 |
| | | | | | | | | |
| Pos. | Master spot no. | Method | Rank | Expect. | Protein information | Cov. | pI | Mass [kDa] |
| 1 | 1127 | Sample 1 | 1 | 0.000 | gi\|72059\|pir\|\|NBHUA2 - leucine-rich alpha-2-glycoprotein - human | 29.2 | 5.7 | 34.56 |
| | | | 2 | 0.385 | gi\|47077177\|dbj\|BAD18510.1 - unnamed protein product [Homo sapiens] | 9.4 | 9.8 | 87.74 |
| 2 2 | 1162 | Sample 2 | - | - | - | - | - | - |
| 3 | 1260 | Sample 3 | 1 | 0.000 | gi\|114006\|sp\|P06727\|APA4_HUMAN -Apolipoprotein A-IV precursor (Apo-AIV) | 38.6 | 5.3 | 45.35 |
| 4 | 1305 | Sample 4 | 1 | 0.000 | gi\|47124562\|gb\|AAH70299.1 - HP protein [Homo sapiens] | 31.7 | 8.8 | 31.65 |
| | | | 2 | 0.031 | gi\|539627\|pir\|\|A46546 - leukocyte common antigen long splice form precursor - human | 7.3 | 5.7 | 148.81 |
| 5 | 1386 | Sample 5 | 1 | 0.362 | gi\|4838009\|gb\|AAD30796.1 - immunoglobulin heavy chain variable region [Homo sapiens] | 52.5 | 8.8 | 13.46 |
| | | | 2 | 0.088 | gi\|4838009\|gb\|AAD30796.1 -immunoglobulin heavy chain variable region [Homo sapiens] | 52.5 | 8.8 | 13.46 |
| | | | 3 | 0.379 | gi\|544379\|sp\|P35574\|GDE_RABIT -Glycogen debranching enzyme (Glycogen debrancher) [Includes: 4-alpha-gLucanotransferase (Oligo-1,4-1,4-glucantransferase); Amylo-alpha-1,6-glucosidase (Amylo-1,6-glucosidase) (Dextrin 6-alpha-D-glucosidase)] | 7.8 | 6.4 | 179.92 |
| 6 | 1400 | Sample 6 | 1 | 0.062 | gi\|15099973\|gb\|AAK84185.1 - anti-thrombospondin immunoglobulin heavy chain variable region [Homo sapiens] | 43.6 | 9.1 | 12.77 |
| 7 | 1439 | Sample 7 | 1 | 0.002 | gi\|22218654\|pdb\|1GPZ\|B - Chain B, The Crystal Structure Of The Zymogen Catalytic Domain Of Complement Protease C1r | 24.8 | 6.6 | 46.14 |
| 8 | 1529 | Sample 8 | 1 | 0.000 | gi\|17318569\|ref\|NP_006112.2 - keratin 1; Keratin-1; cytokeratin 1; hair alpha protein [Homo sapiens] | 17.5 | 8.3 | 66.22 |
| | | | 2 | 0.080 | gi\|386854\|gb\|AAA36153.1 - type II keratin subunit protein | 13.2 | 5.3 | 52.94 |
| | | | 3 | 0.238 | gi\|1346640\|sp\|P35580\|MYHA_HUMAN -Myosin heavy chain, nonmuscle type B (Cellular myosin heavy chain, type B) (Nonmuscle myosin heavy chain-B) (NMMHC-B) gi\|11276948\|pir\|\|A59252 myosin heavy chain, nonmuscle, form IIB - human gi\|641958\|gb\|AAA99177.11 non-muscle myosin B | 5.9 | 5.4 | 229.95 |
| 9 | 1620 | Sample 9 | 1 | 0.318 | gi\|15099973\|gb\|AAK84185.1- anti-thrombospondin immunoglobulin heavy chain variable region [Homo sapiens] | 43.6 | 9.1 | |
| 10 | 1621 | Sample 10 | 1 | 0.457 | gi\|40789364\|dbj\|BAA34505.2 - KIAA0785 protein [Homo sapiens] | 9.3 | 5.7 | 79.96 |
| 11 | 1743 | Sample 11 | 1 | 0.136 | gi\|41406064\|ref\|NP_005955.1-myosin, heavy polypeptide 10, non-muscle; myosin heavy chain, nonmuscle type B; cellular myosin heavy chain, type B type B [Homo sapiens] | 6.3 | 5.4 | 229.95 |
| | | | 2 | 0.169 | gi\|15099973\|gb\|AAK84185.1-anti-thrombospondin immunoglobulin heavy chain variable region [Homo sapiens] | 43.6 | 9.1 | 12.77 |
| 12 | 1827 | Sample 12 | 1 | 0.325 | gi\|28466999\|ref\|NP_787057.1-ARG99 protein [Homo sapiens] | 9.9 | 9.3 | 88.25 |
| | | | 2 | 0.228 | gi\|50255295\|gb\|EAL18030.1 -hypothetical protein CNBK0510 [Cryptococcus neoformans var. neoformans B-3501A] | 9.5 | 5.2 | 159.99 |
| | | | 3 | 0.446 | gi\|404108\|dbj\|lBAA03864.1 - plasma glutathione peroxidase [Homo sapiens] | 35.9 | 9.2 | 16.75 |
| 13 | 1875 | Sample 13 | 1 | 0.239 | gi\|18044197\|gb\|AAH20197.1 - 1-aminocyclopropane-1-carboxylate synthase [Homo sapiens] | 10.8 | 6.0 | 57.89 |
| 14 | 1884 | Sample 14 | - | - | - | - | - | - |
| 15 | 1918 | Sample 15 | 1 | 0.038 | gi\|15099973\|gb\|AAK84185.1-anti-thrombospondin immunoglobulin heavy chain variable region [Homo sapiens] | 43.6 | 9.1 | 12.77 |
| | | | 2 | 0.285 | gi\|546095\|gb\|AAB30327.1 - anti-histone H1 WRI-170 antibody heavy chain variable region [human, systemic lupus erythematosus (Wri) patient, Peptide Partial, 98 aa] | 58.2 | 9.5 | 10.83 |
| 16 | 1919 | Sample 16 | 1 | 0.000 | gi\|339685\|gb\|AAA61181.1 - tr-ansthyretin, | 62.4 | 5.3 | 12.83 |
| | | | 2 | 0.365 | gi\|4365215\|emb\|CAE45949.1 -hypothetical protein [Homo sapiens] | 11.1 | 6.6 | 80.67 |

| | | | | | | | | |
|---|---|---|---|---|---|---|---|---|
| *Cov. (coverage) indicates the percentage of peptides that were identified by MALDI-ToF mass spectroscopy for each of the indicated proteins. | | | | | | | | |

### Example 3

### Proteins Present at Significantly Different Levels in Sera From AMD Patients Compared to Controls

Sera more than 50 AMD patients and age-matched control individuals, representing more than 20 paired experiments, were analyzed by 2-dimensional difference gel electrophoresis (DICE) followed by protein identification by MALDI-ToF mass spectroscopy as described in Example 1, for which examples are provided in Example 2. Proteins that were identified as being present at elevated levels in sera from AMD patients compared to control individuals are listed in Tables 4 and 5 below. Proteins that were identified as being present at reduced levels in sera from AMD patients compared to control individuals are listed in Table6 below.

**Table 4**

| IDENTIFICATION OF PROTEINS PRESENT AT ELEVATED LEVELS IN SERA FROM AMD PATIENTS AS COMPARED TO CONTROLS (P < 0.05) | | | | |
|---|---|---|---|---|
| | | Avg. | | Mass |
| Protein ID | Protein Name* | Ratio | pI | (kDa) |
| gi\|178779\|gb\|AAA51748.1 | Apolipoprotein A-IV³ | 2.31 | 5.2 | 43.37 |
| gi\|3337390\|gb\|AAC27432.1 | Haptoglobin² | 2.25 | 6.1 | 38.73 |
| gi\|182440\|gb\|AAB59530.1 | Fibrinogen gamma prime chain | 1.6 | 5.3 | 52.11 |
| gi\|223002\|prf∥0401173A | Fibrin beta² | 1.59 | 8.3 | 51.37 |

| | | | | |
|---|---|---|---|---|
| *Proteins in this table were classified according to their structure and/or function: ¹complement related protein; ²immune related protein; ³structural protein; ⁴enzyme; ⁵protein of unknown or undetermined function. | | | | |

**Table 5**

| IDENTIFICATION OF PROTEINS PRESENT AT ELEVATED LEVELS IN SERA FROM AMD PATIENTS AS COMPARED TO CONTROLS (P < 0.1 AND > 0.05) | | | | |
|---|---|---|---|---|
| | | Avg. | | Mass |
| Protein ID | Protein Name* | Ratio | pI | (kDa) |
| gi\|4558178\|pdb\|1QAB\|D | Retinol Binding Protein² | 2.57 | 5.5 | 12.98 |
| P0101 | Alpha-1 antichymotrypsin³ | 1.41 | | |
| NP_000925.1 | Alpha-2 antiplasmin³ | 1.41 | | |

| | | | | |
|---|---|---|---|---|
| *Proteins in this table were classified according to their structure and/or function: ¹complement related protein; ²immune related protein; ³structural protein; ⁴enzyme; ⁵protein of unknown or undetermined function. | | | | |

**Table 6**

| IDENTIFICATION OF PROTEINS PRESENT IN SIGNIFICANTLY REDUCED LEVELS IN SERA FROM AMD PATIENTS AS COMPARED TO CONTROLS (P < 0.05) | | | | |
|---|---|---|---|---|
| Protein ID | Protein Name* | Avg. Ratio | pI | Mass (kDa) |
| gi\|7023232\|dbj\|BAA91891.1 | Unnamed protein product⁵ | 37.3 | 6.6 | 84.79 |
| gi\|28373620\|pdb\|MA9\|A | Vitamin D binding protein² | 37.3 | 5.2 | 52.81 |
| gi\|40786791\|gb\|AAR89906.1 | Complement component 3¹ | 16.11 | 6 | 188.67 |
| gi\|5174525\|ref\|NP_005900.1 | Microtubule-associated protein 1B isoform 1³ | 16.11 | 4.7 | 271.8 |
| g\|8928566\|sp\|Q02952\|AK12_ HUMAN | A-kinase anchor protein 12 (AKAP 250) (Myasthenia gravis autoantigen gravin)³ | 16.1 | 4.4 | 191.99 |
| gi\|22218654\|pdb\|1GPZ\|B | Complement component 1, r subcomponent¹ | 4.84 | 6.6 | 46.14 |
| gi\|15099973\|gb\|AAK84185.1 | Ig heavy chain variable region (anti-thrombospondin)² | 4.71 | 9.1 | 12.77 |
| gi\|41406064\|ref\|NP_005955.1 | Myosin, heavy polypeptide 10, non-muscle³ | 2.73 | 5.4 | 229.95 |
| gi\|7318569\|ref\|NP_006112.2 | Keratin 1³ | 2.47 | 8.3 | 66.22 |
| gi\|386854\|gb\|AAA36153.1 | Keratin type II subunit protein³ | 2.47 | 5.3 | 52.94 |
| gi\|1346640\|sp\|P35580\|MYHA_H UMAN | Myosin heavy chain, nonmuscle type B³ | 2.47 | 5.4 | 229.95 |
| gi\|22761659\|db\|BAC11646.1 | Unnamed protein produce⁵ | 2.45 | 5.6 | 24.91 |
| gi\|544379\|sp\|P35574\|GDE_ RABIT | Glycogen debranching enzyme (Glycogen debrancher)⁴ | 2.25 | 6.4 | 179,92 |
| gi\|4838009\|gb\|AAD30796.1 | Ig heavy chain variable rsgion² | 2.25 | 8.8 | 13.46 |
| gi\|1154664\|emb\|CAA62603.1 | Ataxia telangectasia mutated (A-T)³ | 2.24 | 6.4 | 159.31 |
| gi\|34365215\|emb\|CAE45949.1 | TNN13-interacting kinase (FPGT-encoded)⁴ | 2.17 | 6.6 | 80.67 |
| gi\|339685\|gb\|AAA61181.1 | Transthyretin³ | 2.17 | 5.3 | 12.83 |
| gi\|31615331\|pdb\|1HK3\|A | Serum Albumin (mutant R218p)³ | 2.11 | 5.6 | 68.39 |
| gi\|18044197\|gb\|AAH20197.1 | 1-aminocyclopropane-1-carboxylate synthase⁴ | 1.94 | 6 | 57.89 |
| gi\|546095\|gb\|AAB30327.1 | Ig heavy chain variable region (anti-histone H1 WRI-170 antibody) | 1.88 | 9.5 | 10.83 |
| gi\|51476396\|emb\|CAH18188.1 P06727 | Alpha-2 macroglobulin³ Apolipoprotein A-IV³ | 1.78 1.77 | 6 | 164.72 |
| gi\|51467959\|ref\|XP_497224.1 | BMS1-like (similar to KIAA0187; ribosomal)³ | 1.77 | 9.6 | 59.06 |
| gi\|39573703\|dbj\|BAC19850.2 | complement component 1, r subcomponent¹ | 1.77 | 5.9 | 81.72 |
| gi\|23273927\|gb\|AAH35014.1 | NudC domain containing 3 (KIAA1068 protein)³ | 1.77 | 5.1 | 40.88 |
| gi\|34526199\|dbj\|BAC85198.1 | Unnamed protein product⁵ | 1.77 | 7.9 | 54.34 |
| P25311 | Zinc alpha-2 glycoprotein³ | 1.77 | | |
| gi\|28466999\|ref\|NP_787057.1 | ARG99 protein³ | 1.76 | 9.3 | 88.25 |
| gi\|404108\|dbj\|BAA03864.1 | Glutathione peroxidase⁴ | 1.76 | 9.2 | 16.75 |
| gi\|50255295\|gb\|EAL18030.1 | Hypothetical protein CNBK0510⁵ | 1.76 | 5.2 | 159.99 |
| gi\|7428606\|pir∥MHHUM | Ig mu chain C region, membrane-bound splice form² | 1.74 | 5.8 | 52.43 |
| gi\|38649048\|gb\|AAH62986.1 | ZFR protein³ | 1.74 | 9.3 | 69.18 |
| gi\|37790798\|gb\|AAR03501.1 | Angiotensinogen, member 8³ | 1.72 | 5.9 | 53.39 |
| gi\|24308400\|rsf\|NP_612366.1 | Chromosome 10 open reading frame 42⁵ | 1.72 | 9.1 | 40.13 |
| gi\|14625439\|dbj\|BAB61902.1 | KIAA1774 protein⁵ | 1.71 | 4.6 | 126.87 |
| gi\|47124562\|gb\|AAH70299.1 | Haptoglobin² | 1.63 | 8.8 | 31.65 |
| gi\|539627\|pir∥A46546 | Leukocyte common antigen long splice form² | 1.63 | 5.7 | 148.81 |
| gi\|4557225\|ref\|NP_000005.1 | Alpha-2 macroglobulin³ | 1.62 | 6 | 164.69 |
| gi\|14600217\|gb\|AAK71298.1 | TCR beta chain Vbeta13S3² | 1.61 | 5.7 | 16.65 |
| gi\|114006\|sp\|P06727\|APA⁴_ HUMAN | Apolipoprotein A-IV⁴ | 1.57 | 5.3 | 45.35 |
| gi\|40788364\|dbj\|BAA34505.2 | KIAA0785 protein⁵ | 1.57 | 5.7 | 79.96 |
| gi\|7023207\|dbj\|BAA91880.1 | BTB and kelch domain containing 4 protein³ | 1.56 | 5.4 | 59.05 |
| gi\|862457\|dbj\|BAA03941.1 | Enoyl-CoA hydratase/3-hydroxyacyl-CoA dehydrogenase alpha-subunit of trifunctional protein⁴ | 1.56 | 9.4 | 83.68 |
| gi\|7022921\|dbj\|BAA91769.1 | LEPREL1 protein³ | 1.56 | 5 | 61.05 |
| gi\|10437767\|dbj\|BAB15104.1 | Zinc finger protein 2³ | 1.56 | 9.9 | 46.47 |
| gi\|21071030\|ref\|NP_570602.2 | Alpha 1B glycoprotein³ | 1.55 | 5.6 | 54.81 |
| gi\|28207863\|emb\|CAD62585.1 | Alpha-1 antitrypsin³ | 1.55 | 5.1 | 41.6 |
| gi\|51471047\|ref\|XP_370690.3 | AT rich interactive domain 2 (ARID, RFX-like)³ | 1.55 | 9.3 | 215.12 |
| gi\|6470150\|gb\|AAF13605.1 | BiP protein³ | 1.55 | 5.2 | 71.03 |
| gi\|11138513\|gb\|AAG31421.1 | FcRn alpha chain² | 1.55 | 5.9 | 39.72 |
| gi\|10120958\|pdb\|1EXU\|A | MHC-related Fc Receptor² | 1.55 | 6.1 | 30.01 |
| gi\|37747855\|gb\|AAH59367.1 | Transferrin³ | 1.55 | 7.1 | 79.34 |
| gi\|339486\|gb\|AAA61148.1 | Transferrin3 | 1.55 | 9.1 | 16 |
| gi\|5817245\|emb\|CAB53743.1 | dJ20N2.2 (novel protein similar to tubulin, beta polypeptide 4, member Q (TUBB4Q)³ | 1.54 | 4.4 | 18.9 |
| gi\|51476755\|emb\|CAH18343.1 | Hypothetical protein⁵ | 1.54 | 5.9 | 81.7 |
| gi\|50363219\|ref\|NP_001002236.1 | Alpha-1 antitrypsin, member 1³ | 1.52 | 5.4 | 46.89 |
| gi\|28566306\|gb\|AAO43053.1 | Heat shock regulated-1³ | 1.52 | 6.1 | 224.89 |
| gi\|7428607\|pir∥MHHU | Ig mu chain C region, secreted splice form² | 1.52 | 6.4 | 50.01 |
| gl\|1703025\|sp\|P01009 | Alpha-1 antitrypsin³ | 1.5 | 5.4 | 46.89 |
| gi\|72059\|pir∥NBHUA2 | Leucine-rich alpha-2-glycoprotein³ | 1.5 | 5.7 | 34.56 |
| gi\|33469917\|ref\|NP_877423.1 | Minichromosome maintenance protein 4³ | 1.5 | 6.3 | 97.11 |
| gi\|6650772\|gb\|AAF22007.1 | Serotransferrin³ | 1.5 | 7 | 65.33 |
| gi\|553788\|gb\|AAA61141.1 | Transferrin³ | 1.5 | 6 | 55.23 |
| gi\|47077177\|dbj\|BAD18510.1 | ZNF43 variant 3³ | 1.5 | 9.8 | 87.74 |

| | | | | |
|---|---|---|---|---|
| *Proteins in this table were classified according to their structure and/or function: ¹complement related protein; ²immune related protein; ³structural protein; ⁴enzyme; ⁵protein of unknown or undetermined function. | | | | |

Notably, a number of proteins associated with immune-mediated and inflammatory pathways, and drusen biogenesis (see Table 7), confirming previous observations from this laboratory.

**Table 7**

| INFLAMMATION-RELATED AND DRUSEN PROTEINS DIFFERENTIALLY EXPRESSED IN SERA FROM AMD PATIENTS COMPARED TO CONTROLS | | |
|---|---|---|
| Protein | Chromosome | Functional System |
| C1r | 12p13 | Complement |
| C3/C3a | 19p13 | Complement |
| ApoE | 9q13 | Complement |
| IgG HV | 14q32 | Immune |
| IgM | 14q32 | Immune |
| CD45 | 1q32 | Immune |
| Hp | 16q22 | Acute Phase Protein |
| VDBP | 4q123 | Acute Phase Protein |
| A1AT | 14q32 | Anti-elastase |
| A2MG | 12p13 | Collagenase |
| A2AP | 17p12 | Fibrinolysis |

Based upon the data generated, we sought to confirm the differential expression of C3a, a potent proinflammatory by-product of complement activation, in a larger sample set. We analyzed plasma derived from 20 patients with AMD and 20 age-matched control subjects using FACS (BD) flow cytometry. The mean concentration (pg/ml) of C3a was significantly higher (p<0.003) in the AMD patient plasma set, confirming the data obtained using DIGE (Figure 7).

Although the present invention has been described in detail with reference to specific embodiments, those of skill in the art will recognize that modifications and improvements are within the scope and spirit of the invention, as set forth in the claims which follow. All publications and patent documents cited herein are incorporated herein by reference as if each such publication or document was specifically and individually indicated to be incorporated herein by reference. Citation of publications and patent documents (patents, published patent applications, and unpublished patent applications) is not intended as an admission that any such document is pertinent prior art, nor does it constitute any admission as to the contents or date of the same. The invention having now been described by way of written description, those of skill in the art will recognize that the invention can be practiced in a variety of embodiments and that the foregoing description is for purposes of illustration and not limitation of the following claims.

Preferred embodiments of the present invention are disclosed below and are designated as embodiment E1 to embodiment E24.
E1. A method for diagnosing age-related macular degeneration (AMD) in an individual, comprising:
   (a) determining levels of at least two AMD-associated protein markers (biomarkers) in a sample from the individual; and
   (b) comparing the levels of the at least two biomarkers in the sample to reference levels of the at least two biomarkers characteristic of a control population of individuals without AMD,
   wherein a difference in the levels of the at least two biomarkers between the sample from the individual and the control population indicates that the individual has an increased likelihood of having AMD,
   wherein at least one of the biomarkers is other than a complement related protein, and
   wherein at least one of the biomarkers is a complement related protein expressed at elevated levels in individuals with AMD, a complement related protein expressed at decreased levels in individuals with AMD, an immune related protein expressed at elevated levels in individuals with AMD, an immune related protein expressed at decreased levels in individuals with AMD, a structural protein expressed at elevated levels in individuals with AMD, a structural protein expressed at decreased levels in individuals with AMD, an enzyme expressed at elevated levels in individuals with AMD, an enzyme expressed at decreased levels in individuals with AMD, an immune related protein expressed at decreased levels in individuals with AMD, or an immune related protein expressed at decreased levels in individuals with AMD.
E 2. A method for assessing the efficacy of a treatment for age-related macular degeneration (AMD) in an individual, comprising:
   (a) determining levels of at least two biomarkers in a sample from the individual either before treatment or at a first time point after treatment with an agent; and
   (b) determining levels of the at least two biomarkers in a sample from the individual at a later time point during treatment or after treatment with the agent,
   wherein a difference in the levels of the at least two biomarkers measured in (b) compared to (a) in which the levels of the at least two biomarkers moves closer to reference levels of the biomarkers characteristic of a control population of individuals without AMD indicates that the treatment is effective,
   wherein at least one of the biomarkers is other than a complement related protein, and
   wherein at least one of the biomarkers is a complement related protein expressed at elevated levels in individuals with AMD, a complement related protein expressed at decreased levels in individuals with AMD, an immune related protein expressed at elevated levels in individuals with AMD, an immune related protein expressed at decreased levels in individuals with AMD, a structural protein expressed at elevated levels in individuals with AMD, a structural protein expressed at decreased levels in individuals with AMD, an enzyme expressed at elevated levels in individuals with AMD, an enzyme expressed at decreased levels in individuals with AMD, an immune related protein expressed at decreased levels in individuals with AMD, or an immune related protein expressed at decreased levels in individuals with AMD.
E 3. The method of E 1 or 2, wherein at least one of the biomarkers is a complement related protein expressed at elevated levels in individuals with AMD.
E 4. The method of E 1 or 2, wherein at least one of the biomarkers is a complement related protein expressed at decreased levels in individuals with AMD
E 5. The method of E 1 or 2, wherein at least one of the biomarkers is an immune related protein expressed at elevated levels in individuals with AMD.
E 6. The method of E 1 or 2, wherein at least one of the biomarkers is an immune related protein expressed at decreased levels in individuals with AMD.
E 7. The method of E 1 or 2, wherein at least one of the biomarkers is a structural protein expressed at elevated levels in individuals with AMD.
E 8. The method of E 1 or 2, wherein at least one of the biomarkers is a structural protein expressed at decreased levels in individuals with AMD.
E 9. The method of E 1 or 2, wherein at least one of the biomarkers is an enzyme expressed at elevated levels in individuals with AMD.
E 10. The method of E 1 or 2, wherein at least one of the biomarkers is an enzyme expressed at elevated levels in individuals with AMD.
E 11. The method of E 1, 2, 5, or 6 wherein the biomarkers are immune related proteins selected from the group consisting of: gi|3337390|gb|AAC27432.1 (Haptoglobin), gi|82440|gb|AAB59530.1 (Fibrinogen gamma prime chain), gi|223002|prf∥0401173A (Fibrin beta), gi|4558178|pdb|1QAB|D (Retinol Binding Protein), gi|28373620|pdb|1MA9|A (Vitamin D binding protein), gi|15099973|gb|AAK84185.1 (Ig heavy chain variable region (anti-thrombospondin)), gi|4838009|gb|AAD30796.1 (Ig heavy chain variable region), gi|546095|gb|AAB30327.1 (Ig heavy chain variable region (anti-histone H1 WRI-170 antibody)), gi|7428606|pir∥MHHUM (Ig mu chain C region, membrane-bound splice form), gi|47124562|gb|AAH70299.1 (Haptoglobin), gi|539627|pir∥A46546 (Leukocyte common antigen long splice form), gi|14600217|gb|AAK71298.1 (TCR beta chain Vbeta13S3), gi|11138513|gb|AAG31421.1 (FcRn alpha chain), gi|10120958|pdb|1EXU|A (MHC-related Fc Receptor), and gi|7428607|pir∥MHHU (Ig mu chain C region, secreted splice form).
E 12. The method of E 1, 2, 7, or 8 wherein the biomarkers are structural proteins selected from the group consisting of: gi|178779|gb|AAA51748.1 (Apolipoprotein A-IV), P01011 (Alpha-1 antichymotrypsin), NP_000925.1 (Alpha-2 antiplasmin), gi|5174525|ref|NP_005900.1 (Microtubule-associated protein 1B isoform 1), gi|8928566|sp|Q02952|AK12_HUMAN (A-kinase anchor protein 12 (AKAP 250) (Myasthenia gravis autoantigen gravin)), gi|41406064|ref|NP_005955.1 (Myosin, heavy polypeptide 10, non-muscle), gi|17318569|ref|NP_006112.2 (Keratin 1), gi|386854|gb|AAA36153.1 (Keratin type II subunit protein), gi|1346640|sp|P35580|MYHA_HUMAN (Myosin heavy chain, nonmuscle type B), gi|1154664|emb|CAA62603.1 (Ataxia telangectasia mutated (A-T)), gi|339685|gb|AAA61181.1 (Transthyretin), gi|31615331|pdb|1HK3|A (Serum Albumin (mutant R218p)), gi|51476396|emb|CAH18188.1 (Alpha-2 macroglobulin), P06727 (Apolipoprotein A-IV), gi|51467959|ref|XP_497224.1 (BMS1-like (similar to KIAA0187; ribosomal)), gi|23273927|gb|AAH35014.1 (NudC domain containing 3 (KIAA1068 protein)), P25311 (Zine alpha-2 glycoprotein), gi|28466999|ref|NP_787057.1 (ARG99 protein), gi|38649048|gb|AAH62986.1 (ZFR protein), gi|37790798|gb|AAR03501.1 (Angiotensinogen, member 8), gi|4557225|ref|NP_000005.1 (Alpha-2 macroglobulin), gi|114006|sp|P06727|APA4_HUMAN (Apolipoprotein A-IV), gi|7023207|dbj|BAA91880.1 (BTB and kelch domain containing 4 protein), gi|7022921|dbj|BAA91769.1 (LEPREL1 protein), gi|10437767|dbj|BAB15104.1 (Zinc finger protein 2), gi|21071030|ref|NP_570602.2 (Alpha 1B glycoprotein), gi|28207863|emb|CAD62585.1 (Alpha-1 antitrypsin), gi|51471047|ref|XP_370690.3 (AT rich interactive domain 2 (ARID, RFX-like)), gi|6470150|gb|AAF13605.1 (BiP protein), gi|37747855|gb|AAH59367.1 (Transferrin), gi|339486|gb|AAA61148.1 (Transferrin), gi|5817245|emb|CAB53743.1 (dJ20N2.2 (novel protein similar to tubulin, beta polypeptide 4, member Q (TUBB4Q)), gi|50363219|ref|NP_001002236.1 (Alpha-1 antitrypsin, member 1), gi|28566306|gb|AA043053.1 (Heat shock regulate-1), gi|1703025|sp|P01009 (Alpha-1 antitrypsin), gi|72059|pir|NBHUA2 (Leucine-rich alpha-2-glycoprotein), gi|33469917|ref|NP_877423.1 (Minichromosome maintenance protein 4), gi|6650772|gb|AAF22007.1 (Serotransferrin), gi|553788|gb|AAA61141.1 (Transferrin), and gi|47077177|dbj|BAD18510.1 (ZNF438 variant 3).
E 13. The method of E 1, 2, 9, or 10 wherein the biomarkers are enzymes selected from the group consisting of: gi|544379|sp|P35574|GDE_RABIT (Glycogen debranching enzyme (Glycogen debrancher)), gi|34365215|emb|CAE45949.1 (TNN13-interacting kinase (FPGT-encoded)), gi|18044197|gb|AAH20197.1 (1-aminocyclopropane-1-carboxylate synthase), gi|404108|dbj|BAA03864.1 (Glutathione peroxidase), and gi|862437|dbj|BAA03941.1 (Enoyl-CoA hydratase/3-hydroxyacyl-CoA dehydrogenase alpha-subunit of trifunctional protein).
E 14. The method of E 1 or 2, wherein the biomarkers are proteins selected from the group consisting of: gr|7023232|dbj|BAA91891.1 (Unnamed protein product), gi|22761659|dbj|BAC11646.1 (Unnamed protein product), gi|50255295|gb|EAL18030.1 gi|34526199|dbj|BAC85198.1 (Unnamed protein product), (Hypothetical protein CNBK0510), gi|24308400|ref|NP_612366.1 (Chromosome 10 open reading frame 42), gi|14625439|dbj|BAB61902.1 (KIAA1774 protein), gi|40788364|dbj|BAA34505.2 (KIAA0785 protein), and gi|51476755|emb|CAH18343.1 (Hypothetical protein).
E 15. A method for diagnosing age-related macular degeneration (AMD) in an individual, comprising determining a level of an AMD-associated protein marker (biomarker) in a sample from the individual and comparing the level of the biomarker in the sample from the individual to a reference level of the biomarker characteristic of a control population of individuals without AMD, where a difference in the level of the biomarker between the sample from the individual and the control population indicates that the individual has an increased likelihood of having or developing AMD.
E 16. A method for assessing the efficacy of a treatment for age-related macular degeneration (AMD) in a individual, comprising:
   (a) determining a baseline level of an AMD-associated protein marker (biomarker) in a sample from the individual before treatment or at a first time point after treatment; and
   (b) determining the levels of the biomarker in a sample from the individual at a later time point during treatment or after treatment,
   wherein a difference in the level of the biomarker measured in (b) compared to (a) in which the level of the biomarker moves closer to a reference level of the biomarkers characteristic of a control population of individuals without AMD indicates that the treatment is effective.
E 17. The method of E 16, wherein the individual is treated with an agent effective to treat the disease.
E 18. A method for assessing the efficacy of treatment of age-related macular degeneration (AMD) in a individual, comprising:
   (a) determining a baseline level of an AMD-associated protein marker (biomarker) in a sample from the individual at a first time point during a course of treatment with an agent; and
   (b) determining the level of the biomarker in a sample from the individual at a second later time point during or after treatment with the agent,
   wherein a difference in the level of the biomarker measured in (b) compared to (a) in which the level of the biomarker moves closer to a reference level of the biomarkers characteristic of a control population of individuals without AMD indicates that the treatment is effective.
E 19. A method for assessing the efficacy of treatment of age-related macular degeneration (AMD) in an individual, comprising comparing a level of an AMD-associated protein marker (biomarker) in a sample from the individual after administration of an agent to a level of the biomarker in a sample from the individual taken at an earlier time point and to a reference level of the biomarker characteristic of a control population of individuals without AMD, wherein a reduced difference between the level of the biomarker in the individual after administration of the agent compared to the reference level and the level of the biomarker in the individual taken at an earlier time point compared to the reference level indicates that the treatment is effective.
E 20. The method of E 15, 16, 17, 18, or 19, wherein the level of the biomarker is measured by 2-dimensional difference gel electrophoresis (DIGE).
E 21. The method of E 15,16,17,18, or 19, wherein the sample is from the blood, serum, plasma, or urine of the individual.
E 22. The method of E 5, 16, 17, 18, or 19, wherein the levels of a set of biomarkers is determined.
E 23. The method of E 22, wherein the set of biomarkers comprises at least, 2, at least 3, at least 4, or at least 5 of the biomarkers listed in Tables 4 and 5.
E 24. The method of E 22, wherein the set of biomarkers comprises at least, 2, at least 3, at least 4, or at least 5 of the biomarkers listed in Table 6.

## Claims

1. A method for diagnosing age-related macular degeneration (AMD) in an individual, comprising:
(a) determining levels of one or more AMD-associated protein markers (biomarkers) in a sample from the individual; and
(b) comparing the levels of the one or more biomarkers in the sample to reference levels of the one or more biomarkers characteristic of a control population of individuals without. AMD,
wherein a difference in the levels of the one or more biomarkers between the sample from the individual and the control population indicates that the individual has an increased likelihood of having AMD,
wherein at least one of the biomarkers is a complement related protein expressed at elevated levels in individuals with AMD, a complement related protein expressed at decreased levels in individuals with AMD, an immune related protein expressed at elevated levels in individuals with AMD, an immune related protein expressed at decreased levels in individuals with AMD, a structural protein expressed at elevated levels in individuals with AMD, a structural protein expressed at decreased levels in individuals with AMD, an enzyme expressed at elevated levels in individuals with AMD, an enzyme expressed at decreased levels in individuals with AMD, an immune related protein expressed at decreased levels in individuals with AMD, or an immune related protein expressed at decreased levels in individuals with AMD.

2. A method for assessing the efficacy of a treatment for age-related macular degeneration (AMD) in an individual, comprising:
(a) determining levels of one or more biomarkers in a sample from the individual either before treatment or at a first time point after treatment with an agent; and
(b) determining levels of the one or more biomarkers in a sample from the individual at a later time point during treatment or after treatment with the agent,
wherein a difference in the levels of the one or more biomarkers measured in (b) compared to (a) in which the levels of the one or more biomarkers moves closer to reference levels of the biomarkers characteristic of a control population of individuals without AMD indicates that the treatment is effective,
wherein at least one of the biomarkers is a complement related protein expressed at elevated levels in individuals with AMD, a complement related protein expressed at decreased levels in individuals with AMD₅ an immune related protein expressed at elevated levels in individuals with AMD, an immune related protein expressed at decreased levels in individuals with AMD, a structural protein expressed at elevated levels in individuals with AMD, a structural protein expressed at decreased levels in individuals with AMD, an enzyme expressed at elevated levels in individuals with AMD, an enzyme expressed at decreased levels in individuals with AMD, an immune related protein expressed at decreased levels in individuals with AMD, or an immune related protein expressed at decreased levels in individuals with AMD.

3. The method of claims 1 or 2, wherein at least two biomarkers are detected and at least one of the biomarkers is a complement related protein expressed at elevated or decreased levels in individuals with AMD.

4. The method of claims 1 or 2, wherein at least, two biomarkers are detected and at least one of the biomarkers is an immune related protein expressed at elevated or decreased levels in individuals with AMD.

5. The method of claims 1 or 2, wherein at least one of the biomarkers is a structural protein or is an enzyme expressed at elevated or decreased levels in individuals with AMD.

6. The method of claims 1, 2, or 4, wherein at least one biomarker is a protein expressed at decreased levels in individuals with AMD and is gi|28373620|pdb|MA9|A (Vitamin D binding protein).

7. The method of claims 1, 2 or 4, wherein the biomarkers are immune related proteins selected from the group consisting of: gi|28373620|pdb|MA91A (Vitamin D binding protein), gi|3337390|gb|AAC27432.1 (Haptoglobin), gi|182440|gb|AAB59530.1 (Fibrinogen gamma prime chain), gi|223002|prf|0401173A (Fibrin beta), gi|45581781pdb|1QAB|D (Retinol Binding Protein), gi|15099973|gb|AAK84185.1 (Ig heavy chain variable region (anti-thrombospondin)), gi|4838009|gb|AAD30796.1 (Ig heavy chain variable region), gi|546095|gb|AAB30327.1 (Ig heavy chain variable region (anti- histone H1 WRI- 170 antibody)), gi|7428606|pir|MHHUM (Ig mu chain C region, membrane- bound splice form), gi|47124562|gb|AAH70299.1 (Haptoglobin), gi|539627|pir||A46546 (Leukocyte common antigen long splice form), gi|14600217|gb|AAK71298.1 (TCR beta chain Vbeta13S3), gi|11138513|gb|AAG31421.1 (FcRn alpha chain), gi|0120958|pdb|1EXU|A (MHC-related Fc Receptor), gi|22218654|pdb|1GPZ|B (C1r chain B) and gi|7428607]pir||MHHU (Ig mu chain C region, secreted splice form).

8. The method of claims 1, 2 or 5, wherein the biomarkers are structural proteins selected from the group consisting of: gi|178779|gb|AAA51748.1 (Apolipoprotein A-IV)₃ POIOI1 (Alpha- 1 antichymotrypsin), NP_000925.1 (Alpha-2 antiplasmin), gi|5174525 jref|NP_005900.1 (Microtubule-associated protein IB isoform 1), gi|8928566|sp|Q02952|AK12_HUMAN (A-ldnase anchor protein 12 (AKAP 250) (Myasthenia gravis autoantigen gravin)), gi|41406064|ref|NP_00.5955.1 (Myosin, heavy polypeptide 10, non-muscle), gi|17318569|ref|NP_006112.2 (Keratin 1), gi|386854|gb|AAA36153.1 (Keratin type II subunit protein), gi|1346640|sp|P35580|MYHA_HUMAN (Myosin heavy chain, nonmuscle type B), gi|1154664|emb|CAA62603.1 (Ataxia telangectasia mutated (A-T)), gi|339685|gb|AAA61181.1 (Transthyretin), gi|31615331|pdb|1HK3|A (Serum Albumin (mutant R218ρ)), gi|51476396|emb|CAH18188.1 (Alpha-2 macro globulin), P06727 (Apolipoprotein A∼IV), gi|51467959 |reflXP_497224.1 (BMS1-like (similar to KIAAO1 87; ribosomal)), gi|23273927|gb|AAH35014.1 (NudC domain containing 3 (KIAA1068 protein)), P25311 (Zinc alρha-2 glyeoprotein), gi|28466999|ref|NP_787057.1 (ARG99 protein), gi|38649048|gb|AAH62986.1 (ZFR protein), gi|37790798|gb|AAR03.501.1 (Angiotensinogen, member 8), gi|4557225|ref)NP_000005.1 (Alρha-2 macroglobulin), gi|114006|sp|P06727|APA4_HUMAN (Apolipoprotein A-IV), gi|7023207|dbj|BAA91880.1 (BTB and kelch domain containing 4 protein), gi|7022921|dbj|BAA91769.1 (LEPREL1 protein), gi|10437767|dbj|BAB15104.1 (Zinc finger protein 2), gi|21071030|ref|NP_570602.2 (Alpha IB glycoprotein), gi|28207863|emb|CAD62585.1 (Alpha-1 antitrypsin), gi|51471047|ref|XP_370690.3 (AT rich interactive domain 2 (ARID, RFX-like)), gi|6470150|gb|AAF13605.1 (BiP protein), gi|37747855|gb|AAH59367.1 (Transferrin), gi|33394861gb)AAA61148.1 (Transferrin), gi|5817245)emb|CAB53743.1 (dJ20N2.2 (novel protein similar to tubulin, beta polypeptide 4, member Q (TUBB4Q)), gi|50363219|ref|NP_001002236.1 (Alpha-1 antitrypsin, member 1), gi|28566306|gb|AAO43053.1 (Heat shock regulated- 1), gi| 1703025 |sp|PO 1009 (Alpha-1 antitrypsin), gi|72059|piii|NBHUA2 (Leucine-rich alpha-2-glycoprotein), gi|33469917|reflNP_877423.1 (Minichromosome maintenance protein 4), gi|6650772|gb|AAF22007.1 (Serotransferrin), gi|53788|gb|AAA61141.1 (Transferrin), and gi|47077177|db|BAD18510.1 (ZNF438 variant 3).

9. The method of claims 1, 2 or 6, wherein the biomarkers are enzymes selected from the group consisting of: gi|544379|sp|P35574|GDE_RABIT (Glycogen debranching enzyme (Glycogen debrancher)), gi|34365215|emb|CAE45949.1 (TNN1 3-interacting kinase (FPGT-encoded)), gi|18044197|gb|AAH20197.1 (1- aminocyclopropane-1-carboxylate synthase), gi|404108|dbj|BAA03864.1 (Glutathione peroxidase), and gi|862457|dbj|BAA03941.1 (Enoyl-CoA hydrarase/S-bydroxyacyl-CoA dehydrogenase alpha-subunit of trifunctional protein).

10. The method of claims. 1 or 2, wherein the biomarkers are proteins selected from the group consisting of: gi|7023232|dbj|BAA91891.1 (Unnamed protein product), gi|22761659|dbj |BAC11646.1 (Unnamed protein product), gi|50255295|gb|EAL18030.1 gi|34526199|dbj|BAC85198.1 (Unnamed protein product), (Hypothetical protein CNBK0510), gi|24308400|ref|NP_612366.1 (Chromosome 10 open reading frame 42), gi|14625439|dbj|BAB61902.1 (KIAA1774 protein), gi|40788364)dbj|BAA34505.2 (KIAA0785 protein), and gi|51476755|emb|CAH18343.1 (Hypothetical protein).

11. A method for assessing the efficacy of treatment of age-related macular degeneration (AMD) in a individual, comprising:
(a) determining a baseline level of an AMD-associated protein marker (biomarker) in a sample from the individual at a first time point during a course of treatment with an agent; and
(b) determining the level of the biomarker in a sample from the individual at a second later time point during or after treatment with the agent,
wherein a difference in the level of the biomarker measured in (b) compared to (a) in which the level of the biomarker moves closer to a reference level of the biomarkers characteristic of a control population of individuals without AMD indicates that the treatment is effective,

12. The method of claim 11, wherein the sample is the blood, serum, plasma, or urine of the individual.

13. The method of claim 1 or 2, wherein the biomarkers comprises at least, 2, at least 3, at least 4, or at least 5 of the biomarkers listed in Tables 4 and 5.

14. The method of claim 1 or 2, wherein the biomarkers comprises at least, 2, at least 3, at least 4, or at least 5 of the biomarkers listed in Table 6.

15. The method of claim 1 or 2, wherein the biomarkers comprises at least, 2, at least 3, at least 4, or at least 5 of the biomarkers listed in Table 7.
